# EUROPEAN PATENT SPECIFICATION

(11) **EP 3 215 846 B1**
(45) Date of publication and mention of the grant of the patent: **11.03.2020**
(21) Application number: 15857571.2
(22) Date of filing: 05.11.2015
(51) Int. Cl.: G01N 33/533, C12Q 1/00, G01N 21/00

(54) **METAL COMPOSITES FOR ENHANCED IMAGING**
METALLVERBUNDSTOFFE ZUR VERBESSERTEN BILDGEBUNG
COMPOSITES MÉTALLIQUES DESTINÉS À UNE IMAGERIE AMÉLIORÉE

(30) Priority: 05.11.2014 US 201462075785 P; 15.07.2015 US 201562192672 P
(43) Date of publication of application: 13.09.2017
(73) Proprietor: Nirmidas Biotech, Inc., Palo Alto, CA 94303 (US)
(72) Inventor: ROBINSON, Joshua, T., Belmont, CA 94002 (US); TANG, Meijie, Cupertino, CA 95014 (US); ZHAO, Su, Santa Clara, CA 95050 (US); DAI, Hongjie, Cupertino, CA 95014 (US)
(74) Representative: Griffin, Philippa Jane
(86) International application number: PCT/US2015/059321
(87) International publication number: WO 2016/073778

(56) References cited:
- WO-A1-2014/160175
- US-A1- 2003 218 744
- US-A1- 2009 142 847
- US-A1- 2009 305 305
- US-A1- 2012 028 823
- US-A1- 2013 172 207
- US-A9- 2013 102 770
- SCOTT M. TABAKMAN ET AL: "Plasmonic substrates for multiplexed protein microarrays with femtomolar sensitivity and broad dynamic range", NATURE COMMUNICATIONS, vol. 2, 13 September 2011 (2011-09-13), page 466, XP055448196, DOI: 10.1038/ncomms1477
- ANDREW M. FALES ET AL: "Development of Hybrid Silver-Coated Gold Nanostars for Nonaggregated Surface-Enhanced Raman Scattering", JOURNAL OF PHYSICAL CHEMISTRY C, vol. 118, no. 7, 5 February 2014 (2014-02-05), pages 3708-3715, XP055432812, US ISSN: 1932-7447, DOI: 10.1021/jp4091393
- FURTAW MICHAEL D ET AL: "Near-Infrared, Surface-Enhanced Fluorescence Using Silver Nanoparticle Aggregates in Solution", PLASMONICS, SPRINGER US, BOSTON, vol. 9, no. 1, 7 July 2013 (2013-07-07), pages 27-34, XP035940559, ISSN: 1557-1955, DOI: 10.1007/S11468-013-9594-Y [retrieved on 2013-07-07]
- LIANG ET AL.: 'Iron Oxide/Gold Core/Shell Nanoparticles for Ultrasensitive Detection of Carbohydrate-Protein Interactions.' ANALYTICAL CHEMISTRY vol. 81, no. 18, 15 September 2009, pages 7750 - 7756, XP055440366 DOI: 10.1021/AC9012286 Retrieved from the Internet: <URL:http://pubs.acs.org/doi/abs/10.1021/ac 9012286>

## Description

### BACKGROUND

The ability to detect chemical and biological species rapidly with specificity and at very low concentrations is becoming increasingly important, particularly in the environmental, forensic, and medical areas. Detection of low levels pathogenic species, such as agents that pose a biological threat, for example, provides a crucial measure of environmental contamination by such agents since their existence, even at low concentrations, can have serious pathological consequences. Sensitive detection devices therefore, enable the elimination of such pathogens prior to their causing significant harm. There is also a growing need for the rapid and quantitative detection of biological species in a number of biomedical applications, and the healthcare and food industries.

A widely varying set of techniques may be used to detect biological molecules, such as nucleic acids, proteins, antibodies, subcellular vesicles, cells, and tissues, depending on the molecule and application. Fluorescence based detection has become one of the leading sensing technologies in biomedical, biological and related sciences, for example, flow cytometry, gene sequencing, immunoassay, immunohistochemical imaging (IHC) and immunofluorescence imaging, single molecule imaging and fluorescence in situ hybridization (FISH). However, such techniques often encounter problems of insufficient fluorescence signals for high sensitivity, high speed, high signal/noise ratio applications.

Since biological material tends to have high background fluorescence signal in the visible wavelength region, which is reduced in the near-infrared region, detection of the desired biological molecule may be limited, depending on the method, by the intensity of the fluorescence output by the probing fluorescent molecule bound to the biological molecule. For example, for immunoassay techniques such as enzyme-linked immunosorbent assay (ELISA), a common technique for clinical diagnostic measurement of infectious diseases and auto-immune diseases, the detection limit of proteins and antibodies is approximately 1 nanogram/ml (ng/ml) and the dynamic range of ELISA only spans < 3 orders of magnitude. This is sub-optimal considering that clinically relevant levels of biomarkers such as cytokines and antibodies may be well below 1 ng/ml in biological fluids including whole blood, plasma, serum, and saliva at early disease stages, including prostate-specific antigen (PSA) for early stage cancer diagnosis and troponin I (cTnI) for cardiovascular disease. Tabakman, et al. (Nature Communications, 2(3): 466, 2011) described plasmonic substrates for multiplexed protein microarrays with femtomolar sensitivity and broad dynamic range; US 2013/0172207 described fluorescence enhancing plasmonic nanoscopic gold films and assays based thereon; Fales, et al. (Journal of Physical Chemistry, 188(7): 3708-3715, 2014), described the development of hybrid silver-coated gold nanostars for non-aggregated surface-enhanced raman scattering; Furtaw, et al. (Plasmonics, 9(1): 27-34, 2013), described near-infrared, surface-enhanced fluorescence using silver nanoparticle aggregates in solution; WO 2014/160175 described plasmonic substrate for multiplex assessment of type 1 diabetes.

### SUMMARY

In view of the foregoing, there exists a need for detection methodologies that improve the capabilities of fluorescence-based detection schemes for higher signal to background ratios. The methods, systems, and compositions described herein address this need, and provide additional advantages as well.

The present disclosure provides methods and compositions for detecting fluorescent signals with high sensitivity, speed and signal-to-noise ratio. The methods and compositions provided herein may be used for a variety of biomedical imaging and assay applications with fluorescent enhancement in the visible and near-infrared region broadly across about 400 nanometers (nm) to about 2100 nm.

In one aspect, the invention provides a film comprising raised nanostructures on a substrate. The nanostructures of the film comprise silver-on-gold nanoparticles; the nanostructures are separated from one another by gaps; and intensity of a fluorescent signal from a fluorophore in proximity to the film is enhanced relative to the fluorescent signal obtained from the fluorophore in proximity to the substrate in the absence of the film. The gaps have widths between 5 nm to 50 nm, and lengths between 5 nm and 1000 nm. In some embodiments, the nanostructures have an average width and length between 50 nm to 500 nm, such as between 100 nm to 200 nm. In some embodiments, the film has a nanoplate size of between 1000 nm² to 250,000 nm². The film can comprise irregular features and a heterogenous structure. In some embodiments, the height of the film is between 5 nm and 500 nm. The film can be quasi-continuous through a percolating path and conducting based on electron microscopy imaging and electrical conductivity, or discontinuous based on electron microscopy imaging and electrical conductivity. The fluorescent signal that is enhanced can be in the range of 400 nm to 2100 nm. In some embodiments, the fluorescent signal is enhanced for fluorophores within 1000 nm of the surface of the film. In some embodiments, the fluorophore is a near-infra-red fluorophore (e.g. IR680 or IR800) having an emission of about 700 nm to about 800 nm, and the intensity of the fluorescent signal is enhanced by at least 30-fold (e.g. at least 100-fold). In some embodiments, the fluorophore is a visible dye (e.g. DAPI, Alexa488, Cy3, or Cy5) having an emission of about 400 nm to about 700 nm, and the intensity of the fluorescent signal is enhanced by at least 3-fold (e.g. at least 30-fold). The substrate can comprise one or more materials selected from the group consisting of: glass, polystyrene, quartz, silica, nylon, nitrocellulose, polyvinyl chloride, polydimethyl siloxane, polyvinylidene fluoride, polydopamine, silicon, silicon dioxide, a polymer, iron oxide, and a plastic. The substrate can comprise a flat surface, a curved surface, a spherical surface, or a three-dimensional porous membrane. In some cases, the substrate is a bead, such as a bead having a diameter ranging from 0.05 microns to 200 microns. The bead may or may not have a magnetic or paramagnetic core. The bead can be in a container, such as a well in a multi-well plate (e.g. a 96- or 384-well plate). In some cases, the substrate itself is the interior of a well plate, such as a well in a multi-well plate (e.g. a 96- or 384-well plate)

A film of the present invention can further comprise an array of binding elements on the film, wherein the binding elements bind to an analyte. The array can comprise a plurality of different binding elements, each of which binds a different analyte. In some embodiments, the binding elements are selected from the group consisting of: proteins, antibodies, antigen-binding antibody fragments, cells, aptamers, peptides, polynucleotides, exosomes, and tissue slices. The binding elements can be antigens for detecting antibodies in a sample. The antibodies bound by the antigens may be one or more selected from the group consisting of: total human IgG, IgM, IgA and IgE ; anti-HLA (human leukocyte antigen) antibodies; anti-dsDNA antibodies; anti-Smith antibodies; antibodies diagnostic of Systemic Lupus Erythematosus (SLE), such as anti-nucleosome, anti U1RNP, and anti-PO antibodies; antibodies diagnostic of cardiovascular disease (e.g. dilated cardiomyopathy), Toxoplasmosis, Rubella, Rabies, Malaria, lyme disease, African Trypanosomiasis, cholera, cryptosporidiosis, dengue, influenza, Japanese Encephalitis, Leishmaniasis, measles, meningitis, onchocerciasis, pneumonia, tuberculosis, typhoid, or yellow fever; antibodies specific for CMV, HSV-1/2, HBA, HBV, HCV, HDV, HIV; HPV, Ebola virus, rotavirus, human leukocyte antigens, Thyroid Stimulating Hormone Receptor (TSHR), thyroperoxidate, Thyroglobulin, tissue transglutaminase (tTG), endomysium, deamidated gliadin peptide; and antibodies specific for tumor-associated antigens selected from p53, NY-ESO-1, MAGE A4, HuD, CAGE, GBU4-5, and SOX2. The analyte can be a protein, an antibody, a peptide, a nucleic acid, an enzyme, a cell, an exosome, a cell free DNA, or a tissue. The analyte can be a biomarker for a condition of a subject. Examples of analytes include an inflammatory cytokine, a biomarker for cardiovascular disease (e.g. troponin I (cTnI), c-reactive protein (CRP), NT-ProBNP, or an antibody to Annexin A5, SDHA, ATP1A3, titin, myosin, ADBRK, EDNRA, EDNRB, AGTR1, CHRM2, or HSPD), a biomarker for infectious disease (e.g. hepatitis B virus (HBV) core antigen, HBV surface antigen, Dengue NS1 antigen, or antibodies to Toxoplasmosis gondii, Rubella, HCV, HIV, syphilis, or HSV), a biomarker for inflammatory bowel disease, or a biomarker for cancer (e.g. prostate-specific antigen (PSA), carcinoembryonic antigen (CEA), cancer antigen-125 (CA125), AFP, SCC, CA19-9, CA242, NSE, Cyfa21-1, CA15-3, and total T-PSA). In some embodiments, the antigens comprise one or more Toxoplasmosis gondii antigens, one or more Rubella antigens, one or more CMV antigens, and one or more HSV antigens.

In one aspect, the disclosure provides methods for making films of the present disclosure, such as films comprising raised nanostructures comprising silver-on-gold nanoparticles. In some embodiments of the disclosure, the method comprises adsorbing gold (Au) nanoparticle seeds on a substrate, or growing Au nanoparticle seeds in solution or vapor phase on a substrate; and growing silver nanostructures around the gold nanoparticle seeds, such as in the solution phase. The film may have any combination of characteristics of films disclosed herein.

In one aspect, the invention provides a method of detecting an analyte. The method comprises providing a film comprising raised nanostructures on a substrate, wherein nanostructures of the film comprise silver-on-gold nanoparticles, and the nanostructures are separated from one another by gaps as defined in the claims; applying to the film an analyte and a label for the analyte, wherein the label comprises a fluorophore; and detecting the analyte by detecting a fluorescent signal of the fluorophore, wherein intensity of the fluorescent signal is enhanced relative to the fluorescent signal of the fluorophore in the absence of the film. The gaps have widths between 5 nm to 50 nm, and lengths between 5 nm and 200 nm. The film can have any one or more the following characteristics: the nanostructures have an average width and length between 50 nm to 500 nm; the film has a nanoplate size of between 1000 nm² to 250,000 nm²; the height of the film is between 5 nm and 500 nm; the film comprises irregular features and a heterogenous structure; the film imparts a plasmon from about 400 nm to about 2100 nm; the substrate comprises a flat surface, a curved surface, a spherical surface, or a three-dimensional porous membrane; and the substrate is a bead. The fluorescent signal that is enhanced can be in the range of 400 nm to 2100 nm. In some embodiments, the fluorescent signal is enhanced for fluorophores within 1000 nm of the surface of the film. In some embodiments, the fluorophore is a near-infra-red fluorophore (e.g. IR680 or IR800) having an emission of about 700 nm to about 800 nm, and the intensity of the fluorescent signal is enhanced by at least 30-fold (e.g. at least 100-fold). In some embodiments, the fluorophore is a visible dye (e.g. DAPI, Alexa488, Cy3, or Cy5) having an emission of about 400 nm to about 700 nm, and the intensity of the fluorescent signal is enhanced by at least 3-fold (e.g. at least 30-fold). The method can further comprise determining concentration, identity, or location of the analyte based on detecting the fluorescent signal. In some embodiments, (i) the analyte is bound to the label, (ii) the analyte is on a surface (e.g. bound to the surface by a binding element conjugated to the surface), and (iii) step (b) comprises applying the film to the analyte on the surface. The surface can be a DNA microarray, an RNA microarray, a miRNA microarray, a peptide microarray, a protein microarray, an antigen microarray, or an antibody microarray. Imaging can include imaging by a microscope or a scanner. The film can further comprise an array of binding elements on the film, wherein the binding elements bind to the analyte. Binding elements can include any one or more selected from the group consisting of proteins, antibodies, antigen-binding antibody fragments, cells, exosomes, cell free DNA, aptamers, and nucleic acids. In some cases, the binding elements are antigens for detecting antibodies in a sample. The analyte can be a protein, an antibody, a peptide, a nucleic acid, an enzyme, a cell, or a tissue. The analyte can be a biomarker for a condition of a subject. In some cases, the analyte is an inflammatory cytokine, a biomarker for cardiovascular disease (e.g. troponin I, c-reactive protein (CRP), NT-ProBNP, or an antibody to Annexin A5, SDHA, ATP1A3, titin, myosin, ADBRK, EDNRA, EDNRB, AGTR1, CHRM2, or HSPD), a biomarker for infectious disease (e.g. hepatitis B virus (HBV) core antigen, HBV surface antigen, Dengue NS1 antigen, and antibodies to one or more of Toxoplasmosis gondii, Rubella, HCV, HSV, HIV, Syphilis, and CMV) or a biomarker for cancer (e.g. prostate-specific antigen (PSA), carcinoembryonic antigen (CEA), cancer antigen-125 (CA125) AFP, SCC, CA19-9, CA242, NSE, Cyfa21-1, CA15-3, or total T-PSA). The array can comprise a plurality of different binding elements conjugated to the film at known locations, each of which binds a different analyte; and the method further comprises identifying an analyte based on the location of a detected fluorescent signal. In some embodiments, the binding element is an oligonucleotide conjugated to the film, the substrate is a bead, and the analyte is a target polynucleotide that hybridizes to the oligonucleotide via sequence complementarity or an amplification product thereof. Where the binding element is an oligonucleotide, the oligonucleotide can be a primer, and detection can comprise amplifying the target polynucleotide to produce a detectable amplified product. In some embodiments, a plurality of different analytes and a corresponding plurality of different labels are applied to the film, and each of the different labels is detected in a single assay. In some embodiments, the analyte is from a sample of a subject. The method can further comprise identifying a phenotype of a cell in the sample (e.g. cancer) based on detecting the fluorescent signal (e.g. through immunohistochemical (IHC) staining). Detecting can comprise single-molecule imaging and tracking, or single-nanoparticle imaging and tracking. The fluorophore can be a member of a Fluorescence Resonance Energy Transfer (FRET) pair, and fluorescent signals for one or both members of the pair can be enhanced by the film. In some embodiments, the label is a fluorescence in situ hybridization (FISH) probe. Methods of detecting one or more analytes may further comprise diagnosing a subject as having a condition associated with the presence, absence, or level of the one or more analytes, such as a condition described herein. Methods may further comprise taking medical action on the basis of detecting one or more analytes, and/or a resulting diagnosis.

In one aspect, the disclosure provides a method of sequencing a polynucleotide. In some embodiments, the method comprises (a) providing a film comprising raised nanostructures on a substrate, wherein nanostructures of the film comprise silver-on-gold nanoparticles or gold-on-gold nanoparticles, and the nanostructures are separated from one another by gaps; (b) hybridizing an oligonucleotide to a target polynucleotide; (c) extending the oligonucleotide with one or more bases complementary to corresponding positions on the target polynucleotide in the direction of extension; and (d) identifying the one or more bases added in step (c) by detecting a fluorescent signal of one or more fluorophores; wherein intensity of the fluorescent signal is enhanced by the film relative to the fluorescent signal of the fluorophore in the absence of the film. A different fluorophore can be associated with each of four bases. Extension in step (c) can comprise extension by a polymerase, or extension by a ligase. The film may be on a plurality of beads, which may optionally be flowing through or conjugated to a flow cell. The film may have any one or more characteristics of films described herein.

In one aspect, the disclosure provides a composition comprising beads distributed in or on a porous substrate. In some embodiments, the beads have bead surfaces, and on the bead surfaces is a film comprising raised nanostructures; the nanostructures comprise silver-on-gold nanoparticles, or gold-on-gold nanoparticles; the nanostructures are separated from one another by gaps; and intensity of a fluorescent signal from a fluorophore in proximity to the film is enhanced relative to the fluorescent signal obtained from the fluorophore in proximity to the substrate in the absence of the film. The composition may be further characterized by any one or more of the following: (a) the beads have a magnetic core; (b) the beads have a diameter between 0.05 microns to 200 microns; (c) the porous substrate is a porous membrane material; and/or (d) the film comprises a plurality of binding elements on the film. The porous substrate can be a porous membrane material comprising nitrocellulose, glass fiber, nylon, or cellulose acetate. The film may comprise a plurality of binding elements on the film, and the binding elements are selected from the group consisting of proteins, antibodies, antigen-binding antibody fragments, cells, aptamers, and nucleic acids. The film may have any one or more characteristics of films described herein.

In one aspect, the disclosure provides a bead on which is a film comprising raised nanostructures. In some embodiments of the disclosure, the nanostructures comprise silver-on-gold nanoparticles, or gold-on-gold nanoparticles; the nanostructures are separated from one another by gaps; a plurality of fluorophores are conjugated to the bead or to the film; and intensity of a fluorescent signal from the fluorophore is enhanced relative to the fluorescent signal obtained from the fluorophore on the bead in the absence of the film. The bead may further comprise one or more binding element conjugated to the bead or to the film. A plurality of such beads may be employed in a method for labeling a target. The method can comprise contacting the target with a plurality of the beads, wherein the binding element binds to the target. The film may have any one or more characteristics of films described herein. In some embodiments of the disclosure, the bead has one or more of (e.g. all of) the following characteristics: (a) the bead has a size in the range of 0.01-10 microns; (b) the bead comprises a magnetic coating underlying the film, optionally wherein the magnetic coating comprises iron oxide; (c) a plurality of binding elements are complexed to the bead, wherein the binding elements bind an analyte, optionally wherein the analyte is an exosome, a protein, an antibody, a polynucleotide (e.g. DNA or RNA), or a cell-free polynucleotide (e.g. DNA or RNA); and (d) the bead comprises a polymer or silica core.

In one aspect, the disclosure provides a bead on which is a film comprising raised nanostructures. In some embodiments of the disclosure, the nanostructures comprise silver-on-gold nanoparticles, or gold-on-gold nanoparticles; the nanostructures are separated from one another by gaps; a plurality of binding elements are conjugated to the bead or to the film; and intensity of a fluorescent signal from a fluorophore complexed to the bead is enhanced relative to the fluorescent signal obtained from the fluorophore complexed to the bead in the absence of the film. In some embodiments of the disclosure, the bead has one or more of (e.g. all of) the following characteristics: (a) the bead has a size in the range of 0.01-10 microns; (b) the bead comprises a magnetic coating underlying the film, optionally wherein the magnetic coating comprises iron oxide; (c) a plurality of binding elements are complexed to the bead, wherein the binding elements bind an analyte, optionally wherein the analyte is an exosome, a protein, an antibody, a polynucleotide (e.g. DNA or RNA), or a cell-free polynucleotide (e.g. DNA or RNA); and (d) the bead comprises a polymer or silica core. The film may have any one or more characteristics of films described herein. In some embodiments of the disclosure, the film has one or more of the following characteristics: the gaps have widths between 5 nm to 50 nm, and lengths between 5 nm and 200 nm; the nanostructures have an average width and length between 50 nm to 500 nm; the film has a nanoplate size of between 1000 nm² to 250,000 nm²; the height of the film is between 5 nm and 500 nm; the film comprises irregular features and a heterogenous structure; and the film imparts a plasmon from about 400 nm to about 2100 nm. A plurality of such beads may be employed in a method of detecting an analyte, such as an exosome or a polynucleotide (e.g. cell-free DNA). In some embodiments of the disclosure, the method comprises (a) combining the beads with a sample comprising the analyte, wherein the analyte binds to the binding element; (b) labeling the bound analyte with a label that binds the analyte, wherein the label comprises a fluorophore; and (c) detecting the bound analyte by detecting a fluorescent signal from the fluorophore; wherein intensity of the fluorescent signal is enhanced relative to the fluorescent signal of the fluorophore in the absence of the film. In some embodiments of the disclosure, (a) the analyte is an exosome and the label is an antibody that binds a surface antigen of the exosome; or (b) the analyte is a cell-free DNA and the label is an intercalating dye.

In one aspect, the disclosure provides an apparatus comprising a film on a film substrate, and an analyte receptacle. In some embodiments of the disclosure, (a) the film comprises raised nanostructures, wherein the raised nanostructures (i) comprise silver-on-gold nanoparticles, or gold-on-gold nanoparticles, and (ii) are separated from one another by gaps; (b) the analyte receptacle is configured to retain an analyte in contact with a first surface of the analyte receptacle; (c) the film has a film surface that is in contact with a second surface of the analyte receptacle or is spaced within 1000nm of the first surface of the analyte receptacle, wherein the second surface is located opposite the first surface; and (d) intensity of a fluorescent signal from a fluorophore associated with the analyte is enhanced relative to the fluorescent signal obtained from the fluorophore in the absence of the film. In some embodiments of the disclosure, (a) the film comprises raised nanostructures, wherein the raised nanostructures (i) comprise silver-on-gold nanoparticles, or gold-on-gold nanoparticles, and (ii) are separated from one another by gaps; (b) the film substrate is a bead in the analyte receptacle; (c) the analyte receptacle is configured to retain an analyte and the bead; and (d) intensity of a fluorescent signal from a fluorophore in proximity to the film is enhanced relative to the fluorescent signal obtained from the fluorophore in proximity to the substrate in the absence of the film. Where the apparatus comprises a bead, the apparatus may be further characterized in that: (a) the beads have a magnetic core; (b) the beads have a diameter between 0.05 microns to 200 microns; (c) the film comprises a plurality of binding elements on the film; and/or (d) the analyte receptacle is a well of a multi-well plate. The film can have any one or more of the following characteristics: the gaps have widths between 5 nm to 50 nm, and lengths between 5 nm and 200 nm; the nanostructures have an average width and length between 50 nm to 500 nm; the film has a nanoplate size of between 1000 nm² to 250,000 nm²; the height of the film is between 5 nm and 500 nm; the film comprises irregular features and a heterogenous structure; and the film imparts a plasmon from about 400 nm to about 2100 nm. The apparatus may be provided for use in a method of detecting an analyte, which method may further comprise applying to the analyte receptacle an analyte and a label for the analyte, wherein the label comprises a fluorophore; and detecting the analyte by detecting a fluorescent signal of the fluorophore, wherein intensity of the fluorescent signal is enhanced relative to the fluorescent signal of the fluorophore in the absence of the film. The film may have any one or more characteristics of films described herein.

In one aspect, the disclosure provides a method of detecting one or more antibodies in a sample. In some embodiments of the disclosure, the method comprises: (a) providing a film comprising raised nanostructures on a substrate, wherein (i) nanostructures of the film comprise silver-on-gold nanoparticles or gold-on-gold nanoparticles, (ii) the nanostructures are separated from one another by gaps, and (iii) a plurality of antigens are complexed to a surface of the film; (b) contacting the plurality of antigens with a sample and one or more labels for detecting the one or more antibodies bound to an antigen of the plurality of antigens, wherein each label comprises a fluorophore; and (c) detecting the one or more bound antibodies by detecting a fluorescent signal of the fluorophore, wherein intensity of the fluorescent signal is enhanced relative to the fluorescent signal of the fluorophore in the absence of the film; wherein the plurality of antigens comprises one or more Toxoplasmosis gondii antigens, one or more Rubella antigens, one or more CMV antigens, and one or more HSV antigens. In some embodiments, the plurality of antigens further comprise one or more HIV antigens, and/or one or more Syphilis antigens. In some embodiments, the one or more antibodies are IgG antibodies, and the one or more labels are anti-IgG antibodies. In some embodiments, (i) the one or more antibodies comprise one or more antibody subtypes selected from IgG, IgM, IgA, and IgE; (ii) the one or more labels comprise a plurality of corresponding label antibodies selected from (and optionally includes all of) anti-IgG, anti-IgM, anti IgA, and anti-IgE antibodies; (iii) the fluorophore of a label antibody corresponding to one subtype is different from the fluorophore corresponding to any other subtype (e.g. selected from Cy3, Cy5, IR680, and IR800), and (iv) the method further comprises identifying or quantifying the different subtypes based on the fluorescent signal. In some embodiments, the plurality of antigens are arranged at known locations in an array, and the method further comprises identifying the one or more bound antibodies based on the location of the fluorescent signal in the array. The film and substrate can be any of those described herein. In some embodiments, the film comprises silver-on-gold nanoparticles. In some embodiments, the film has one or more of the following characteristics: the gaps have widths between 5 nm to 50 nm, and lengths between 5 nm and 200 nm; the nanostructures have an average width and length between 50 nm to 500 nm; the film has a nanoplate size of between 1000 nm² to 250,000 nm²; the height of the film is between 5 nm and 500 nm; the film comprises irregular features and a heterogenous structure; the film imparts a plasmon from about 400 nm to about 2100 nm; the substrate comprises a flat surface, a curved surface, a spherical surface, or a three-dimensional porous membrane; and the substrate is a bead. In some embodiments, the fluorophore is a near-infra-red fluorophore having an emission of about 700 nm to about 800 nm, and the intensity of the fluorescent signal is enhanced by at least 30-fold. In some embodiments, the fluorophore is a visible dye having an emission of about 400 nm to about 700 nm, and the intensity of the fluorescent signal is increased by at least 3-fold. In some embodiments, the method further comprises determining concentration, identity, and/or location of the analyte based on detecting the fluorescent signal. In some embodiments, the sample is a whole blood, plasma, serum, saliva, or urine sample having a volume between 1-100 µL. In some embodiments, the sample is a whole blood sample having a volume between 1-10 µL, optionally diluted in a diluent solution to a total volume of 100 µL or less.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** in panels (a)-(e) shows a series of example images showing silver-on-gold film (Ag/Au) formation on glass in accordance with an embodiment.
**FIG. 2** in panels (a)-(c) shows example atomic force microscopy topography images of Au⁰ seeds deposited on SiO₂ with varying initial HAuCl₄ concentrations, in accordance with an embodiment.
**FIG. 3** in panels (a)-(c) shows a series of example images showing the signal enhancement of TOTO-3, a fluorophore with excitation at 640 nm and emission at 660 nm, on example cAg/Au and Au/Au substrates as compared with glass substrates using the Cy5 channel on a genepix scanner from Agilent.
**FIG. 4** in panels (a)-(d) shows a series of example images showing the performance of (cAg/Au) films as substrates for a cytokine sandwich assay, in accordance with an embodiment.
**FIG. 5** in panels (a)-(c) shows a series of example images showing the performance of (cAg/Au) films as a substrate for reverse phase protein assay, in accordance with an embodiment.
**FIG. 6** shows results for enhanced detection of a biomarker by flow cytometry assay using an Ag/Au film in accordance with an embodiment. In both graphs, the x-axis is a logarithmic scale from 10⁻³ to 10⁶, and the y-axis is a logarithmic scale from 10¹ to 10⁷.
**FIG. 7** shows example Toxo IgG pilot testing on plasmonic slides (B) as compared to dye test results (A).
**FIG. 8** shows example Toxo IgM pilot testing on plasmonic slides (B) as compared to ELISA results (A). In view (A), the eight tallest bars correspond to positive sera, while the others correspond to negative sera. In view (B), all bars correspond to matched results.

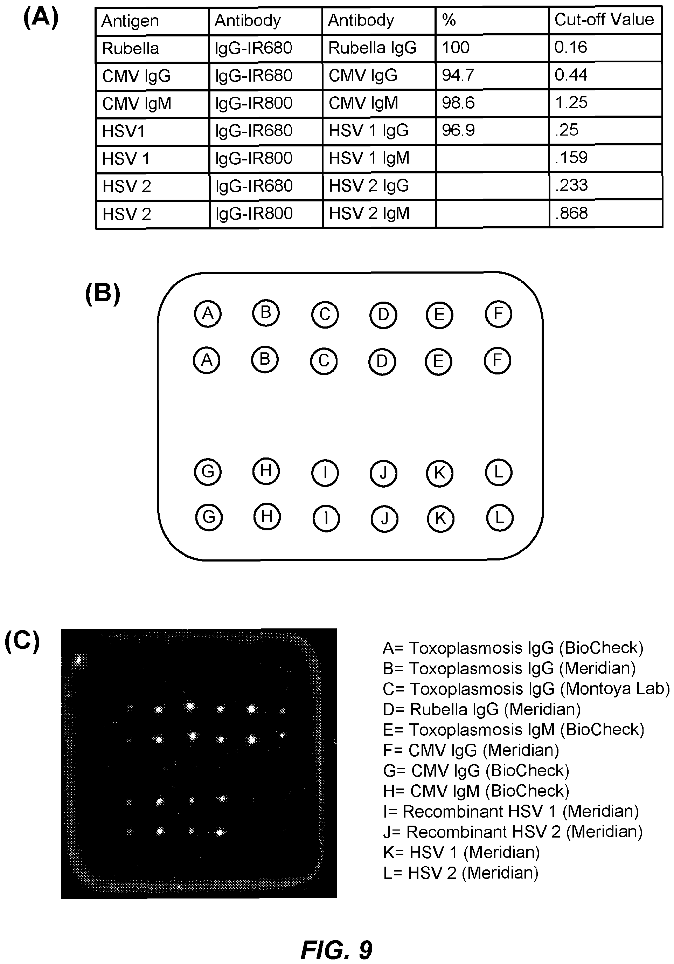

**FIG. 9** shows example results of ToRCH pilot testing on plasmonic slides in accordance with an embodiment. Antigens, antibodies detected, and antibodies for labeling are indicated in (A). A fluorescent image of detection results are provided in (C). A schematic layout indicating the identity of the printed antigens in spots of the fluorescent image is provided in (B), which indicates that spots A, B, C, D, E, F, G, H, I, J, K, and L correspond to antigens bound by Toxoplasmosis IgG (BioCheck), Toxoplasmosis IgG (Meridian), Toxoplasmosis IgG (Montoya Lab), Rubella IgG (Meridian), Toxoplasmosis IgG (Biocheck), CMV IgG (Meridian), CMV IgG (Biocheck), CMV IgM (Biocheck), recombinant HSV1 (Meridian), recombinant HSV2 (Meridian), HSV1 (Meridian), and HSV2 (Meridian), respectively.
**FIG. 10** shows example IgD detection in saliva and whole blood on a plasmonic slide, in accordance with an embodiment.

### DETAILED DESCRIPTION

As used herein, the singular form "a", "an" and "the" include plural references unless the context clearly dictates otherwise. For example, the term "a cell" includes a plurality of cells, including mixtures thereof.

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. A nucleotide analog may be an analog or mimic of a primary nucleotide having modification on the primary nucleobase (A, C, G, T and U), the deoxyribose/ribose structure, the phosphate group of the primary nucleotide, or any combination thereof. For example, a nucleotide analog can have a modified base, either naturally existing or man-made. Examples of modified bases include, without limitation, methylated nucleobases, modified purine bases (e.g. hypoxanthine, xanthine, 7-methylguanine, isodG), modified pyrimidine bases (e.g. 5,6-dihydrouracil and 5-methylcytosine, isodC), universal bases (e.g. 3-nitropyrrole and 5-nitroindole), non-binding base mimics (e.g. 4-methylbezimidazole and 2,4-diflurotoluene or benzene), and no base (abasic nucleotide where the nucleotide analog does not have a base). Examples of nucleotide analogs having modified deoxyribose (e.g. dideoxynucleosides such as dideoxyguanosine, dideoxyadenosine, dideoxythymidine, and dideoxycytidine) and/or phosphate structure (together referred to as the backbone structure) includes, without limitation, glycol nucleotides, morpholinos, and locked nucleotides.

As used herein, the term "fluorescence" refers to the process wherein a molecule relaxes to its ground state from an excited state by emission of a photon. As used herein, fluorescence can also encompass phosphorescence. For fluorescence, a molecule is promoted to an electronically excited state generally by the absorption of ultraviolet, visible, or near infrared radiation. The excited molecule then decays back to the ground state, or to a lower-lying excited electronic state, by emission of light.

As used herein, the term "plasmonically active" in reference to a material refers to a material which supports plasmons (e.g., surface plasmons), thereby exhibiting plasmonic properties. Surface plasmons may be used to enhance the surface sensitivity of several spectroscopic measurements including fluorescence, Raman scattering, and second harmonic generation.

As used herein, the term "plasmonic properties" refers to properties exhibited by surface plasmons, or the collective oscillations of electrical charge on the surfaces of metals. In this sense, plasmonic properties refers to measurable properties, such as properties described in Nagao et al. "Plasmons in nanoscale and atomic-scale systems," Sci. Technol. Adv. Mater. 11 (2010) 054506 (12pp), describing plasmonic sensors, such as those used for surface-enhanced IR absorption spectroscopy (SEIRA), surface-enhanced Raman scattering (SERS). Another plasmonic property is plasmon-enhanced fluorescence, described e.g. in Sensors and Actuators B 107 (2005) 148-153.

As used herein, the term "continuous" refers to the inter-connectivity of nanostructures in plasmonic metal film, creating an electrically conductive path, optionally with gaps existing between some of the nanostructures that are not in the conducting path.

As used herein, the term "discontinuous" refers to the presence of one or more isolated nanostructures in plasmonic metal film, where the nanostructures are separated from each other and not interconnected.

As used herein, the term "proximity" refers to a distance between a fluorescent molecule and nanostructures in plasmonic metal film, within which distance the fluorescence intensity of the fluorescent molecule increases, such as by a specified fold-increase. In some cases, proximity may be measured in angstroms (e.g. within 1-1000 angstroms), in nanometers (e.g. within 1-1000 nm), or millimeters (e.g. within 1-10 mm). In some cases, fluorescent intensity is enhanced for fluorophores within about 1000 nm of the surface of the film.

### Compositions and Systems

In some of the various aspects, provided herein are compositions, systems and methods that are capable of amplifying or enhancing fluorescent signals, such as signals spanning from about 400 nm to about 2100 nm. The compositions and systems may be able to facilitate fluorescence detection with improved detection limits, sensitivity, speed, specificity and signal-to-noise ratio, thereby finding use in a wide context of applications that rely on fluorescence as a detection method.

In one aspect, the present disclosure provides a film comprising raised nanostructures on a substrate. The film may be further characterized in that the nanostructures of the film comprise silver-on-gold nanoparticles; the nanostructures are separated from one another by gaps; and intensity of a fluorescent signal from a fluorophore in proximity to the film is enhanced relative to the fluorescent signal obtained from the fluorophore in proximity to the substrate in the absence of the film. The film may be applied directly or indirectly to the substrate. For certain applications, the nanostructures comprise a first metal on nanoparticles of a second metal, where the first and second metal may be the same or different (e.g. gold-on-gold, or silver-on-gold). The nanostructures may be formed by at least one type of noble metals, for example, ruthenium (Ru), rhodium (Rh), palladium (Pd), silver (Ag), osmium (Os), iridium (Ir), platinum (Pt), and gold (Au). The nanostructures can take various shapes, e.g., sphere, cube, cuboid, cone, cylinder, prism, pyramid, tube, plate, disc, rod, or any regular or irregular shapes. In some cases, the nanostructures comprise nanoparticles. In cases where more than one type of metals are included in the nanostructures, the metals can form a layered or core/shell structure, for example, a silver-on-gold nanoparticle. In some cases, each of the nanostructures has the same shape. In some cases, it may be preferred to have nanostructures of different shapes (or heterogeneous).

Sizes (e.g., length, width, height etc.) of the nanostructures may vary, depending upon, applications that the nanostructures are used for. For example, it may be preferred to have nanostructures that are much smaller than the wavelength of light used for fluorescence excitation and emission. In some cases, the nanostructures may have a width, length, and/or height of less than or equal to about 1 millimeter (mm), such as less than or equal to about 750 micron (µm), 500 µm, 250 µm, 100 µm, 75 µm, 50 µm, 25 µm, 10 µm, 5 µm, 1 µm, 900 nm, 800 nm, 700 nm, 600 nm, 500 nm, 400 nm, 300 nm, 200 nm, 100 nm, 90 nm, 80 nm, 70 nm, 60 nm, 50 nm, 40 nm, 30 nm, 20 nm, 10 nm, 9 nm, 8 nm, 7 nm, 6 nm, 5 nm, 4 nm, 3 nm, 2 nm, 1 nm, 800 picometers (pm), 600 pm. 400 pm, 200 pm, 100 pm, 75 pm, 50 pm. 25 pm or 10 pm. In some cases, the width, length, and/or height of the nanostructures may be greater than or equal to about 1 pm, 5 pm, 10 pm, 25 pm, 50 pm, 75 pm, 100 pm, 250 pm, 500 pm, 750 pm, 1 nm, 2nm, 3 nm, 4 nm, 5 nm, 6nm, 7 nm, 8 nm, 9 nm, 10 nm, 15 nm, 20 nm, 30 nm, 40 nm, 50 nm, 60 nm, 70 nm, 80 nm, 90 nm, 100 nm, 150 nm, 200 nm, 250 nm, 300 nm, 350 nm, 400 nm, 450 nm, 500 nm, 600 nm, 700 nm, 800 nm, 900 nm, 1 µm, 5 µm, 10 µm, 30 µm, 50 µm, 70 µm, 90 µm, 110 µm, 130 µm, 150 µm, 200 µm, 400 µm, 600 µm, 800 µm, or 1mm. In some cases, the width, length, and/or height of the nanostructures may be between any of the two values described herein, for example, the nanostructures may have an average width and length between about 50 nm to about 500 nm, or between about 100 nm to about 200 nm. As a further example, the film may have a height of between 5 nm to 500 nm.

The nanostructures may have a cross-sectional area of at least about 0.001 nm², 0.005 nm², 0.01 nm², 0.05 nm², 0.075 nm², 0.1 nm², 0.5 nm², 0.75 nm², 1 nm², 10 nm², 20 nm², 40 nm², 60 nm², 80 nm², 100 nm², 250 nm², 500 nm², 750 nm², 1,000 nm², 2,500 nm², 5,000 nm², 7,500 nm², 10,000 nm², 25,000 nm², 50,000 nm², 75,000 nm², 100,000 nm², 200,000 nm², 300,000 nm², 400,000 nm², 500,000 nm², 600,000 nm², 700,000 nm², 800,000 nm², 900,000 nm², 1,000,000 nm², 2,500,000 nm², 5,000,000 nm², 7,500,000 nm², or 10,000,000 nm². In some cases, the nanostructures may have a cross-sectional area of less than or equal to about 25,000,000 nm², 10,000,000 nm², 8,000,000 nm², 6,000,000 nm², 4,000,000 nm², 2,000,000 nm², 1,000,000 nm², 800,000 nm², 600,000 nm², 500,000 nm², 450,000 nm², 400,000 nm², 350,000 nm², 300,000 nm², 250,000 nm², 200,000 nm², 150,000 nm², 100,000 nm², 80,000 nm², 60,000 nm², 50,000 nm², 40,000 nm², 30,000 nm², 20,000 nm², 10,000 nm², 8,000 nm², 6,000 nm², 4,000 nm², 2,000 nm², 1,800 nm², 1,600 nm², 1,400 nm², 1,200 nm², 1,000 nm², 900 nm², 800 nm², 700 nm², 600 nm², 500 nm², 400 nm², 300 nm², 200 nm², 100 nm², 90 nm², 80 nm², 70 nm², 60 nm², 50 nm², 40 nm², 30 nm², 20 nm², 10 nm², 8 nm², 6 nm², 4 nm², 2 nm², 1 nm², 0.75 nm², 0.5 nm², 0.25 nm², 0.1 nm², 0.075 nm², 0.05 nm², 0.025 nm², 0.01 nm², 0.0075 nm², 0.005 nm², 0.0025 nm², 0.001 nm², 0.0005 nm², or 0.0001nm². In some cases, the cross-sectional area of the nanostructures may fall between any of the two values described herein, for example, between about 100 nm² to about 250,000 nm², or between about 1,000 nm² to about 250,000 nm².

In some cases, each of the nanostructures comprised in the film may have the same shape, structure, and/or size. In some cases, the nanostructures may be of varied size, shape, and/or structure. Depending on the application, it may be preferred that a certain percentage of the nanostructures have the same or a different size, shape, and/or structure, for example, about 1%, 5%, 10%, 15%, 20%, 30%, 40%, 50%, 60%, 70%, 75%, 80%, 85%, 90%, 91%, 92%, 93%, 94%, 95%, 96%, 97%, 98%, 99%, or 100% of the nanostructures may have the same or a different size, shape, and/or structure.

Nanostructures may or may not be separated from each other. In cases where the nanostructures are separated from one another, they may be separated by gaps. Gap distance, a distance between the nanostructures, may vary. In some cases, a large gap distance may be created. In other cases, a small gap distance may be used. Large and small gaps can be constructed at different locations within the same film. In some cases, the gap distances or an average gap distance may be less than or equal to about 1 mm, such as less than or equal to about 750 µm, 500 µm, 250 µm, 100 µm, 75 µm, 50 µm, 25 µm, 10 µm, 7.5 µm, 5 µm, 2.5 µm, 1 µm, 900 nm, 800 nm, 700 nm, 600 nm, 500 nm, 400 nm, 300 nm, 200 nm, 100 nm, 90 nm, 80 nm, 70 nm, 60 nm, 50 nm, 40 nm, 30 nm, 20 nm, 10 nm, 9 nm, 8 nm, 7 nm, 6 nm, 5 nm, 4 nm, 3 nm, 2 nm, 1 nm, 0.75 nm, 0.5 nm, 0.25 nm, 0.1 nm, 0.075 nm, 0.05 nm, 0.025 nm, 0.01 nm, 0.0075 nm, 0.005 nm, 0.0025 nm, or 0.001 nm. In some cases, the gap distance may be at least about 0.0001 nm, 0.0005 nm, 0.001 nm, 0.005 nm, 0.01 nm, 0.05 nm, 0.1 nm, 0.5 nm, 1 nm, 5 nm, 7.5 nm, 10 nm, 20 nm, 40 nm, 60 nm, 80 nm, 100 nm, 200 nm, 300 nm, 400 nm, 500 nm, 600 nm, 700 nm, 800 nm, 900 nm, 1 µm, 2 µm, 3 µm, 4 µm, 5 µm, 6 µm, 7 µm, 8 µm, 9 µm, 10 µm, 25 µm, 50 µm, 75 µm, 100 µm, 500 µm and 750 µm. In some cases, the gap distance can be between any of the two values described herein, for example, between about 1 nm and about 1,000 nm.

Gaps may be of varies widths and/or lengths. For example, the gaps may have widths and/or lengths that are less than or equal to about 5,000 nm, 4,000 nm, 3,000 nm, 2,000 nm, 1,000 nm, 800 nm, 600 nm, 400 nm, 200 nm, 100 nm, 80 nm, 60 nm, 40 nm, 20 nm, 10 nm, 7.5 nm, 5 nm, 1 nm, 0.75 nm, 0.5 nm, 0.25 nm, 0.1 nm, 0.05 nm, 0.01 nm, 0.005 nm, or 0.001 nm. In some cases, the widths and/or lengths of the gaps may be greater than or equal to about 0.005 nm, 0.0075 nm, 0.01 nm, 0.05 nm, 0.075 nm, 0.1 nm, 0.5 nm, 0.75 nm, 1 nm, 2.5 nm, 5 nm, 7.5 nm, 10 nm, 30 nm, 50 nm, 70 nm, 90 nm, 100 nm, 200 nm, 300 nm, 400 nm, 500 nm, 750 nm, 1,000 nm, 2,500 nm, or 5,000 nm. In some cases, the gaps may have widths and/or lengths falling into a range between any of the two values described herein, for example, the gaps may have widths between about 5 nm to about 50nm, and lengths between about 5 nm to about 200 nm.

As provided herein, a film can be continuous, quasi-continuous, or discontinuous, depending upon, the inter-connectivity of nanostructures comprised in the film. For example, the film may be a continuous film, such that it comprises raised nanostructures that are interconnected, creating an electrically conductive path, with gaps existing between some of the nanostructures that are not in the conducting path. By contrast, the film may be discontinuous, such that at least some portion of the film (e.g. at least 10%, 25%, 50%, 75%, 90%, or more) is comprised of nanostructures that are not connected by an electrically conducting path.

Features (e.g., size, dimension, and/or structure of nanostructures comprised in the film) and characteristics (e.g., roughness, thickness, continuity, electrical conductivity, and/or inter-connection of the nanostructures) of a film as provided in the present disclosure can be determined and characterized by various techniques, such as, for example, electrical conductivity measurement, and/or microscopy techniques including standard light microscopy , transmission electron microscopy (TEM), confocal laser scanning microscopy, scanning electron microscopy (SEM) and atomic force microscopy (AFM). For example, a film that is quasi-continuous through a percolating path and conducting can be determined based on electron microscopy imaging and/or electrical conductivity. In some examples, a discontinuous film may be characterized based on electron microscopy imaging and electrical conductivity.

As provided herein, various materials may be used to fabricate a substrate. For example, the materials can be organic or inorganic, synthetic or natural, solid or semi-solid. Non-limiting examples of materials that can be used to form the substrate may comprise glass, quartz, plastic, nitrocellulose, silicon-based material (e.g., silicon, silicon dioxide), polymer (e.g., polystyrene, nylon, polydopamine (PDA), polyvinyl chloride (PVC), poly(dimethylsiloxane) (PDMS), polyvinylidene fluoride etc.), bioassay or combinations thereof.

A substrate may be of varied shape, e.g., 3-dimensional or 2-dimensional, regular or irregular, homogeneous or heterogeneous. In some cases, the lateral shape of the substrate can be of round, square, rectangle, polygon, elliptical, elongated bar, polygon, or any other regular or irregular shapes or combinations thereof. For example, in some cases, the substrate is a bead or barcode. The substrate may be a magnetic bead (e.g., a bead comprising a magnetic or paramagnetic core) that may facilitate subsequent separation and detection processes. The substrate may also comprise a flat surface, a curved surface, a spherical surface, or a three-dimensional porous membrane. In some cases, the substrate may be the interior of a well of a multi-well plate, comprised, in some cases, of polystyrene. In some cases, the substrate may be all wells of a multi-well plate.

In some cases, the substrate is a bead. Beads may be made from any of a variety of materials (e.g. a substrate material described herein), and some varieties are commercially available. In some embodiments of the disclosure, the beads have an average diameter of at least about 0.001 microns (e.g. 0.005 microns, 0.01 microns, 0.05 microns, 0.1 microns, 1 micron, 10 microns, 50 microns, 100 microns, 250 microns, 500 microns, or more); less than about 500 microns (e.g. 400 microns, 200 microns, 100 microns, 50 microns, 25 microns, 10 microns, 1 micron, or less); or between any of these (e.g. ranging from about 0.01 microns to about 10 microns, about 0.05 microns to about 500 microns, about 0.1 microns to 200 microns, or about 0.1 microns to about 8 microns). Beads may be provided in a container, such as in a tube or a well of a multi-well plate. As with any of the other substrates described herein, the bead and/or the film on the bead may be conjugated to one or more binding elements, such as multiple copies of a single binding element, or a plurality of different binding elements. In some cases, the beads are further disposed in or on a support, such as a porous substrate material. A variety of porous substrates are available, selection of which may depend on the particular application, the size of the beads, and the like. Non-limiting examples of porous membranes for use as bead supports include nitrocellulose, hydrogels, 3D polymers, glass fiber, nylon, or cellulose acetate.

With the film of the present disclosure, intensity of a fluorescent signal from a fluorescent molecule (e.g., a fluorophore) in proximity to the film may be enhanced relative to the fluorescent signal obtained in the absence of the film. Such enhancement of the fluorescent signal may be characterized or quantified by an enhancement factor, which is defined as the ratio of a fluorescent signal obtained with the presence of the film to the same signal obtained without the film. For example, if a fluorescent signal is 410 and 15, with and without the presence of the film, respectively, then the enhancement factor is about 27. With the aid of a film provided herein, fluorescent signal in the range of 400 nm to 2100 nm can be enhanced with varied enhancement factors, dependent upon, e.g., wavelength of the fluorescent signals, features and characteristics of the film etc. For example, the intensity of a near-infra-red fluorescent signal having an emission of about 700 nm to about 800 nm may be enhanced by about 30-fold or more (e.g. at least 50-fold, 100-fold, 250-fold, 500-fold, or more) by using a film of the present disclosure. In some examples, the intensity of the fluorescent signal of a visible dye having an emission of about 400 nm to about 700 nm may be enhanced by about 3-fold or more (e.g. at least 5-fold, 10-fold, 25-fold, 50-fold, 100-fold, or more). In some cases, the film has fluorescence enhancement of fluorescent signals up to about 1,000-fold, 800-fold, 700-fold, 600-fold, 500-fold, 400-fold, 300-fold, 200-fold, 100-fold, 90-fold, 80-fold, 70-fold, 60-fold, 50-fold, 40-fold, 30-fold, 20-fold, 10-fold, 9-fold, 8-fold, 7-fold, 6-fold, 5-fold, 4-fold, 3-fold, or 2-fold. In some cases, by utilizing the film of the present disclosure, the intensity of the fluorescent signals can be enhanced by at least about 1-fold, 2-fold, 3-fold, 4-fold, 5-fold, 6-fold, 7-fold, 8-fold, 9-fold, 10-fold, 20-fold, 30-fold, 40-fold, 50-fold, 60-fold, 70-fold, 80-fold, 90-fold, 100-fold, 150-fold, 200-fold, 250-fold, 300-fold, 350-fold, 400-fold, 450-fold, 500-fold, 600-fold, 700-fold, 800-fold, 900-fold, or 1,000-fold. In some cases, the enhancement factor may be between any of the two values described herein, for example, about 10- to 30-fold, or about 100-to 200-fold. In some cases, enhancement is obtained for a fluorescent signal having a wavelength of at least 300 nm, 400 nm, 500 nm, 600 nm, 700 nm, 800 nm, 900 nm, 1000 nm, 1200 nm, 1400 nm, 1600 nm, 1800 nm, 2000 nm, or more; less than 2200 nm, 2000 nm, 1800 nm, 1600 nm, 1400 nm, 1200 nm, 1000 nm, 900 nm, 800 nm, 700 nm, 600 nm, 500 nm, or less; or for a range of wavelengths between any of these, such as between 300nm to 2200nm, 400nm to 800nm, 500nm to 900 nm, or 800 nm to 1400 nm.

A wide variety of fluorescent molecules can be utilized in the present disclosure, for example, fluorophores, small molecules, dyes, fluorescent proteins and quantum dots. Non-limiting examples of fluorescent molecules may include: fluorescent in situ hybridization(FISH) probes, 1,5 IAEDANS; 1,8-ANS; 4-Methylumbelliferone; 5-carboxy-2,7-dichlorofluorescein; 5-Carboxyfluorescein (5-FAM); 5-Carboxynapthofluorescein; 5-Carboxytetramethylrhodamine (5-TAMRA); 5-FAM (5-Carboxyfluorescein); 5-HAT (Hydroxy Tryptamine); 5-Hydroxy Tryptamine (HAT); 5-ROX (carboxy-X-rhodamine); 5-TAMRA (5-Carboxytetramethylrhodamine); 6-Carboxyrhodamine 6G; 6-CR 6G; 6-JOE; 7-Amino-4-methylcoumarin; 7-Aminoactinomycin D (7-AAD); 7-Hydroxy-4-methylcoumarin; 9-Amino-6-chloro-2-methoxyacridine; ABQ; Acid Fuchsin; ACMA (9-Amino-6-chloro-2-methoxyacridine); Acridine Orange; Acridine Red; Acridine Yellow; Acriflavin; Acriflavin Feulgen SITSA; Aequorin (Photoprotein); AFPs-AutoFluorescent Protein-(Quantum Biotechnologies); Alexa Fluor 350™; Alexa Fluor 430™; Alexa Fluor 488™; Alexa Fluor 532™; Alexa Fluor 546™; Alexa Fluor 568™; Alexa Fluor 594™; Alexa Fluor 633™; Alexa Fluor 647™; Alexa Fluor 660™; Alexa Fluor 680™; Alizarin Complexion; Alizarin Red; Allophycocyanin (APC); AMC, AMCA-S; AMCA (Aminomethylcoumarin); AMCA-X; Aminoactinomycin D; Aminocoumarin; Aminomethylcoumarin (AMCA); Anilin Blue; Anthrocyl stearate; APC (Allophycocyanin); APC-Cy7; APTRA-BTC; APTS; Astrazon Brilliant Red 4G; Astrazon Orange R; Astrazon Red 6B; Astrazon Yellow 7 GLL; Atabrine; ATTO-TAG™ CBQCA; ATTO-TAG™ FQ; Auramine; Aurophosphine G; Aurophosphine; BAO 9 (Bisaminophenyloxadiazole); BCECF (high pH); BCECF (low pH); Berberine Sulphate; Beta Lactamase; Bimane; Bisbenzamide; Bisbenzimide (Hoechst); bis-BTC; Blancophor FFG; Blancophor SV; BOBO™-1; BOBO™-3; Bodipy 492/515; Bodipy 493/503; Bodipy 500/510; Bodipy 505/515; Bodipy 530/550; Bodipy 542/563; Bodipy 558/568; Bodipy 564/570; Bodipy 576/589; Bodipy 581/591; Bodipy 630/650-X; Bodipy 650/665-X; Bodipy 665/676; Bodipy FI; Bodipy FL ATP; Bodipy FI-Ceramide; Bodipy R6G SE; Bodipy TMR; Bodipy TMR-X conjugate; Bodipy TMR-X, SE; Bodipy TR; Bodipy TR ATP; Bodipy TR-X SE; BO-PRO™-1; BO-PRO™-3; Brilliant Sulphoflavin FF; BTC; BTC-SN; Calcein; Calcein Blue; Calcium Crimson™; Calcium Green; Calcium Green-1 Ca.sup.2+Dye; Calcium Green-2 Ca.sup.2+; Calcium Green-SN Ca.sup.2+; Calcium Green-C18 Ca.sup.2.sup.+; Calcium Orange; Calcofluor White; Carboxy-X-rhodamine (5-ROX); Cascade Blue™; Cascade Yellow; Catecholamine; CCF2 (GeneBlazer); CFDA; Chlorophyll; Chromomycin A; Chromomycin A; CL-NERF; CMFDA; Coumarin Phalloidin; C-phycocyanine; CPM Methylcoumarin; CTC; CTC Formazan; Cy2™; Cy3.1 8; Cy3.5™; Cy3™; Cy5.1 8; Cy5.5™; Cy5™; Cy7™; cyclic AMP Fluorosensor (FiCRhR); Dabcyl; Dansyl; Dansyl Amine; Dansyl Cadaverine; Dansyl Chloride; Dansyl DHPE; Dansyl fluoride; DAPI; Dapoxyl; Dapoxyl 2; Dapoxyl 3' DCFDA; DCFH (Dichlorodihydrofluorescein Diacetate); DDAO; DHR (Dihydrorhodamine 123); Di-4-ANEPPS; Di-8-ANEPPS (non-ratio); DiA (4-Di-16-ASP); Dichlorodihydrofluorescein Diacetate (DCFH); DiD-Lipophilic Tracer; DiD (DiIC18(5)); DIDS; Dihydrorhodamine 123 (DHR); Dil (DiIC18(3)); Dinitrophenol; DiO (DiOC18(3)); DiR; DiR (DiIC18(7)); DM-NERF (high pH); DNP; Dopamine; DTAF; DY-630-NHS; DY-635-NHS; ELF 97; Eosin; Erythrosin; Erythrosin ITC; Ethidium Bromide; Ethidium homodimer-1 (EthD-1); Euchrysin; EukoLight; Europium (III) chloride; EYFP; Fast Blue; FDA; Feulgen (Pararosaniline); FIF (Formaldehyde Induced Fluorescence); FITC; Flazo Orange; Fluo-3; Fluo-4; Fluorescein (FITC); Fluorescein Diacetate; Fluoro-Emerald; Fluoro-Gold (Hydroxystilbamidine); Fluor-Ruby; Fluor X; FM 1-43™; FM 4-46; Fura Red™ (high pH); Fura Red™/Fluo-3; Fura-2; Fura-2/BCECF; Genacryl Brilliant Red B; Genacryl Brilliant Yellow 10GF; Genacryl Pink 3G; Genacryl Yellow 5GF; GeneBlazer (CCF2); Gloxalic Acid; Granular blue; Haematoporphyrin; Hoechst 33258; Hoechst 33342; Hoechst 34580; HPTS; Hydroxycoumarin; Hydroxystilbamidine (FluoroGold); Hydroxytryptamine; Indo-1, high calcium; Indo-1, low calcium; Indodicarbocyanine (DiD); Indotricarbocyanine (DiR); Intrawhite Cf; JC-1; JO-JO-1; JO-PRO-1; LaserPro; Laurodan; LDS 751 (DNA); LDS 751 (RNA); Leucophor PAF; Leucophor SF; Leucophor WS; Lissamine Rhodamine; Lissamine Rhodamine B; Calcein/Ethidium homodimer; LOLO-1; LO-PRO-1; Lucifer Yellow; Lyso Tracker Blue; Lyso Tracker Blue-White; Lyso Tracker Green; Lyso Tracker Red; Lyso Tracker Yellow; LysoSensor Blue; LysoSensor Green; LysoSensor Yellow/Blue; Mag Green; Magdala Red (Phloxin B); Mag-Fura Red; Mag-Fura-2; Mag-Fura-5; Mag-indo-1; Magnesium Green; Magnesium Orange; Malachite Green; Marina Blue; Maxilon Brilliant Flavin 10 GFF; Maxilon Brilliant Flavin 8 GFF; Merocyanin; Methoxycoumarin; Mitotracker Green FM; Mitotracker Orange; Mitotracker Red; Mitramycin; Monobromobimane; Monobromobimane (mBBr-GSH); Monochlorobimane; MPS (Methyl Green Pyronine Stilbene); NBD; NBD Amine; Nile Red; Nitrobenzoxadidole; Noradrenaline; Nuclear Fast Red; Nuclear Yellow; Nylosan Brilliant lavin E8G; Oregon Green; Oregon Green 488-X; Oregon Green™; Oregon Green™ 488; Oregon Green™ 500; Oregon Green™ 514; Pacific Blue; Pararosaniline (Feulgen); PBFI; PE-Cy5; PE-Cy7; PerCP; PerCP-Cy5.5; PE-TexasRed [Red 613]; Phloxin B (Magdala Red); Phorwite AR; Phorwite BKL; Phorwite Rev; Phorwite RPA; Phosphine 3R; PhotoResist; Phycoerythrin B [PE]; Phycoerythrin R [PE]; PKH26 (Sigma); PKH67; PMIA; Pontochrome Blue Black; POPO-1; POPO-3; PO-PRO-1; PO-PRO-3; Primuline; Procion Yellow; Propidium lodid (PL); PyMPO; Pyrene; Pyronine; Pyronine B; Pyrozal Brilliant Flavin 7GF; QSY 7; Quinacrine Mustard; Red 613 [PE-TexasRed]; Resorufin; RH 414; Rhod-2; Rhodamine; Rhodamine 110; Rhodamine 123; Rhodamine 5 GLD; Rhodamine 6G; Rhodamine B; Rhodamine B 200; Rhodamine B extra; Rhodamine BB; Rhodamine BG; Rhodamine Green; Rhodamine Phallicidine; Rhodamine Phalloidine; Rhodamine Red; Rhodamine WT; Rose Bengal; R-phycocyanine; R-phycoerythrin (PE); S65A; S65C; S65L; S65T; SBFI; Serotonin; Sevron Brilliant Red 2B; Sevron Brilliant Red 4G; Sevron Brilliant Red B; Sevron Orange; Sevron Yellow L; SITS; SITS (Primuline); SITS (Stilbene Isothiosulphonic Acid); SNAFL calcein; SNAFL-1; SNAFL-2; SNARF calcein; SNARF1; Sodium Green; SpectrumAqua; SpectrumGreen; SpectrumOrange; Spectrum Red; SPQ (6-methoxy-N-(3-sulfopropyl)quinolinium); Stilbene; Sulphorhodamine B can C; Sulphorhodamine Extra; SYTO 11; SYTO 12; SYTO 13; SYTO 14; SYTO 15; SYTO 16; SYTO 17; SYTO 18; SYTO 20; SYTO 21; SYTO 22; SYTO 23; SYTO 24; SYTO 25; SYTO 40; SYTO 41; SYTO 42; SYTO 43; SYTO 44; SYTO 45; SYTO 59; SYTO 60; SYTO 61; SYTO 62; SYTO 63; SYTO 64; SYTO 80; SYTO 81; SYTO 82; SYTO 83; SYTO 84; SYTO 85; SYTOX Blue; IR680; IR880; IR-26; IR-1051; IR-1061; SYTOX Green; SYTOX Orange; Tetracycline; Tetramethylrhodamine (TRITC); Texas Red™; Texas Red-X™ conjugate; Thiadicarbocyanine (DiSC3); Thiazine Red R; Thiazole Orange; Thioflavin 5; Thioflavin S; Thioflavin TCN; Thiolyte; Thiozole Orange; Tinopol CBS (Calcofluor White); TMR; TO-PRO-1; TO-PRO-3; TO-PRO-5; TOTO-1; TOTO-3; TriColor (PE-Cy5); TRITC TetramethylRodaminelsoThioCyanate; True Blue; TruRed; Ultralite; Uranine B; Uvitex SFC; WW 781; X-Rhodamine; XRITC; Xylene Orange; Y66F; Y66H; Y66W; YO-PRO-1; YO-PRO-3; YOYO-1; YOYO-3, Sybr Green, Thiazole orange (interchelating dyes); Alexa Fluor dye series (such as Alexa Fluor 350, Alexa Fluor 405, 430, 488, 500, 514, 532, 546, 555, 568, 594, 610, 633, 635, 647, 660, 680, 700, and 750); Cy Dye fluorophore series (such as Cy3, Cy3B, Cy3.5, Cy5, Cy5.5, Cy7); Oyster dye fluorophores (such as Oyster-500, -550, -556, 645, 650, 656); DY-Labels series (such as DY-415, -495, -505, -547, -548, -549, -550, -554, -555, -556, -560, -590, - 610, -615, -630, -631, -632, -633, -634, -635, -636, -647, -648, -649, -650, -651, -652, -675, -676, -677, -680, -681, -682, -700, -701, -730, -731, -732, -734, -750, -751, -752, -776, -780, -781, - 782, -831, -480XL, -481XL, -485XL, -510XL, -520XL, -521XL); ATTO fluorescent labels (such as ATTO 390, 425, 465, 488, 495, 520, 532, 550, 565, 590, 594, 610, 611X, 620, 633, 635, 637, 647, 647N, 655, 680, 700, 725, 740); CAL Fluor and Quasar dyes (such as CAL Fluor Gold 540, CAL Fluor Orange 560, Quasar 570, CAL Fluor Red 590, CAL Fluor Red 610, CAL Fluor Red 635, Quasar 670); EviTags or quantum dots of the Qdot series (such as the Qdot 525, Qdot565, Qdot585, Qdot605, Qdot655, Qdot705, Qdot 800); fluorescein, rhodamine, phycoerythrin, or combinations thereof.

In some examples, the fluorescent molecule is a near-infrared fluorophore, such as IR680 or IR880. In general, the term "near-infrared" (NIR) is used to refer to the near infrared region of the electromagnetic spectrum (e.g. from 0.6 to 2.1 µm). Other examples of NIR fluorophores include Cy5, Cy5.5, and Cy7, each of which are available from GE Healthcare; VivoTag-680, VivoTag-S680, VivoTag-S750, each of which are available from VisEn Medical; AlexaFluor660, AlexaFluor680, AlexaFluor700, AlexaFluor750, and Alexa Fluor790, each of which are available from Invitrogen; Dy677, Dy676, Dy682, Dy752, Dy780, each of which are available from Dyomics; DyLight 677, available from Thermo Scientific; HiLyte Fluor 647, HiLyte Fluor 680, and HiLyte Fluor 750, each of which are available from AnaSpec; IRDye 800, IRDye800CW, IRDye 800RS, IRDye680CW and IRDye 700DX, each of which are available from Li-Cor; and ADS780WS, ADS830WS, and ADS832WS, each of which are available from American Dye Source. Also, quantum dots of CdSe, PbS, CuInS₂, rare earth nanoparticles, carbon nanotubes belong to NIR fluorescence agents emitting in the 700-2100nm range. NIR labels can be enhanced by NIR fluorescence enhancement (NIR-FE), whereby nanostructures of the disclosure favorably modify the spectral properties of fluorophores and alleviate some of their more classical photophysical constraints.

In some examples, the fluorescent molecule is a visible dye such as Alexa 488, Cy3 or Cy5. In general, the terms "visible dye" and "visible label" are used to refer to a label with fluorescence emission wavelength in the visible region of the electromagnetic spectrum (e.g. 300 nm to 650 nm). Other examples of visible dyes include Cy3 available from GE Healthcare; FITC available from Pierce; VivoTag-645, available from VisEn Medical; AlexaFluor350, AlexaFluor405, AlexaFluor430, AlexaFluor488, AlexaFluor514, AlexaFluor532, AlexaFluor546, AlexaFluor555, AlexaFluor594, AlexaFluor610, AlexaFluor633 and Alexa Fluor647, each of which are available from Invitrogen; Dy405, Dy415, Dy430, Dy490, Dy495, Dy505, Dy530, Dy547, Dy560, Dy590, Dy605, Dy610, Dy615, Dy630, and Dy647 each of which are available from Dyomics; DyLight547 and DyLight647, each of which are available from Thermo Scientific; HiLyte Fluor 405, HiLyte Fluor 488, HiLyte Fluor 532, HiLyte Fluor 555, and HiLyte Fluor 594, each of which are available from AnaSpec;. Visible labels can be enhanced by fluorescence enhancement (FE), whereby silver-on-gold nanostructures favorably modify the spectral properties of fluorophores and alleviate some of their more classical photophysical constraints.

In some cases, a fluorescent molecule may be a member of a fluorescence resonance energy transfer (FRET) pair and FRET is used to produce a signal that can be correlated with the binding of binding elements and analytes. FRET arises from the properties of certain fluorophores. Such produced signals for one or both members of the pair may be enhanced with the presence of a film as provided herein. Molecules that can be used in FRET may include the fluorophores described above, and include fluorescein, 5-carboxyfluorescein (FAM), 2'7'-dimethoxy-4'5'-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'-dimethylaminophenylazo) benzoic acid (DABCYL), and 5-(2-aminoethyl)aminonaphthalene-1-sulfonic acid (EDANS).

In some cases, the acceptor of the FRET pair is used to quench the fluorescence of the donor. In some cases, the acceptor has little to no fluorescence. The FRET acceptors that are useful for quenching are referred to as quenchers. Quenchers useful in the methods of the present invention include, without limitation, Black Hole Quencher Dyes (Biosearch Technologies such as BHQ-0, BHQ-1, BHQ-2, BHQ-3, BHQ-10; QSY Dye fluorescent quenchers (from Molecular Probes/Invitrogen) such as QSY7, QSY9, QSY21, QSY35, and other quenchers such as Dabcyl and Dabsyl; Cy5Q and Cy7Q and Dark Cyanine dyes (GE Healthcare), which can be used, for example, in conjunction with donor fluorophors such as Cy3B, Cy3, or Cy5; DY-Quenchers (Dyomics), such as DYQ-660 and DYQ-661; and ATTO fluorescent quenchers (ATTO-TEC GmbH), such as ATTO 540Q, 580Q, 612Q.

### Binding elements and analytes

A film of the present disclosure may further comprise a plurality of binding elements. The plurality of binding elements may be an array of binding elements. The array of binding elements may be a microarray. In some embodiments, the plurality of binding elements are in direct or indirect contact with the film, such as by way of direct attachment or indirectly attached to an intermediate that is attached to the film. The binding elements may be on top of the film. The binding elements may be underneath the film. In some cases, the binding elements may be attached to a substrate and indirectly contact the film. For example, the substrate may comprise an avidin or streptavidin layer which is between the film and the binding elements. The binding elements may be attached to the substrate via a linking molecule (or a linker). The linker may be any type of molecule (e.g. chemical or biological) that is capable of linking the binding elements with the substrate. In some cases, the linker is a chemical bond. For example, the binding elements can be attached covalently to the surface of the substrate.

A number of different chemical surface modifiers can be added to substrates to attach the binding elements to the substrates. Examples of chemical surface modifiers may include, but not limited to, N-hydroxy succinimide (NHS) groups, amines, aldehydes, epoxides, carboxyl groups, hydroxyl groups, hydrazides, hydrophobic groups, membranes, maleimides, biotin, streptavidin, thiol groups, nickel chelates, photoreactive groups, boron groups, thioesters, cysteines, disulfide groups, alkyl and acyl halide groups, glutathiones, maltoses, azides, phosphates, phosphines, and combinations thereof. In one cases, substrate surfaces reactive towards amines may be utilized. Examples of such surfaces may include NHS-esters, aldehyde, epoxide, acyl halide, and thio-ester. Molecules (e.g., proteins, peptides, glycopeptides) with free amine groups may react with such surfaces to form covalent bond with the surfaces. Nucleic acid probes with internal or terminal amine groups can also be synthesized, (e.g., from IDT or Operon) and bound (e.g., covalently or non-covalently) to surfaces using similar chemistries.

In some cases, an array of capture agents or binding elements may be attached to a film via an extra layer, for example, a self-assembled monolayer on the film. In some examples, a hydrophilic polymer (e.g., Polyethylene glycol (PEG)) or dextran is linked to a self-assembled monolayer on the film, wherein the binding elements (e.g., biological molecules) are linked to the hydrophilic polymer or dextran.

As provided herein, systems of the present disclosure may further comprise an array of samples including analytes to be detected or identified. The array may be a microarray. The array of samples may be disposed in contact with a film of the present disclosure.

Any substance may be the source of a sample. The sample may be a fluid, e.g., a biological fluid. A fluidic sample may include, but is not limited to, blood or blood component (e.g., whole blood, plasma), cord blood, saliva, urine, sweat, serum, semen, vaginal fluid, gastric and digestive fluid, spinal fluid, placental fluid, cavity fluid, ocular fluid, serum, breast milk, lymphatic fluid, or combinations thereof. Analytes may be detected at low concentration in a sample, or in samples of small volumes. For example, the volume of a sample may be less than 1000 µL, 750 µL, 500 µL, 250 µL, 100 µL, 50 µL, 25 µL, 10 µL, or less. In some embodiments, the fluid sample is between 1-100 µL, or 1-10 µL. Fluid sample may be diluted in a diluent solution (e.g. fetal bovine serum, non-cross-reacting animal serum, or a BSA solution in PBST), such as up to a total volume of 500 µL, 250 µL, 100 µL, or less. A sample may be solid, for example, a biological tissue. The sample may comprise normal healthy tissues. The tissues may be associated with various types of organs. Non-limiting examples of organs may include brain, breast, liver, lung, kidney, prostate, ovary, spleen, lymph node (including tonsil), thyroid, pancreas, heart, skeletal muscle, intestine, larynx, esophagus, stomach, or combinations thereof. A sample may be an environmental sample (e.g. samples from agricultural fields, lakes, rivers, water reservoirs, air vents, walls, roofs, soil samples, plants, or swimming pools), or an industrial sample (e.g. samples from clean rooms, hospitals, food processing areas, food production areas, food stuffs, medical laboratories, pharmacies, or pharmaceutical compounding centers).

A sample may comprise tumors. Tumors may be benign (non-cancer) or malignant (cancer). Non-limiting examples of tumors include : fibrosarcoma, myxosarcoma, liposarcoma, chondrosarcoma, osteogenic sarcoma, chordoma, angiosarcoma, endothelio sarcoma, lymphangiosarcoma, lymphangioendothelio sarcoma, synovioma, mesothelioma, Ewing's tumor, leiomyosarcoma, rhabdomyosarcoma, gastrointestinal system carcinomas, colon carcinoma, pancreatic cancer, breast cancer, genitourinary system carcinomas, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, endocrine system carcinomas, testicular tumor, lung carcinoma, small cell lung carcinoma, non-small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, or combinations thereof. The tumors may be associated with various types of organs. Non-limiting examples of organs may include brain, breast, liver, lung, kidney, prostate, ovary, spleen, lymph node (including tonsil), thyroid, pancreas, heart, skeletal muscle, intestine, larynx, esophagus, stomach, or combinations thereof.

A sample may comprise a mix of normal healthy tissues or tumor tissues. The tissues may be associated with various types of organs. Non-limiting examples of organs include brain, breast, liver, lung, kidney, prostate, ovary, spleen, lymph node (including tonsil), thyroid, pancreas, heart, skeletal muscle, intestine, larynx, esophagus, stomach, or combinations thereof.

In some cases, a sample comprises a variety of cells, including, but not limited to: eukaryotic cells, prokaryotic cells, fungi cells, heart cells, lung cells, kidney cells, liver cells, pancreas cells, reproductive cells, stem cells, induced pluripotent stem cells, gastrointestinal cells, blood cells, cancer cells, bacterial cells, bacterial cells isolated from a human microbiome sample, and circulating cells in the human blood, one or more of which may be the subject of a detection method utilizing a film of the present disclosure. In some cases, the sample comprises contents of a cell, such as, for example, the contents of a single cell or the contents of multiple cells.

Depending upon the specific applications, a binding element as provided herein may be designed or selected to bind to one or more specific analytes with greater affinity than it binds to other substances contained in a sample. The binding between the binding elements and the analytes can be through various types of molecular recognition mechanisms, for example, hybridization. The strength of binding can be referred to as "affinity". Affinities between biological molecules can be influenced by non-covalent intermolecular interactions including, for example, hydrogen bonding, hydrophobic interactions, electrostatic interactions and Van der Waals forces. In some cases, for example, for multiplexed analysis, a plurality of analytes and binding elements are involved. For example, an experiment may involve testing the binding between a plurality of different nucleic acid molecules or between different proteins. In such experiments, analytes may be preferred to bind to binding elements for which they have greater affinity. In some cases, the plurality of binding elements may be configured to conjugate to the film at different known locations and each of the binding element binds to a different analyte. For example, an array may comprise at least 2 (e.g. at least 10, 25, 50, 100, 1000, 5000, 10000, or more) different binding elements, each having binding specificity for a different analyte (e.g. a different polynucleotide sequence, or a different protein). Based on the location of a detected fluorescent signal, the analyte can be identified.

The binding can be, for example, a receptor-ligand, enzyme-substrate, antibody-antigen, or a nucleic acid hybridization interaction. The binding element/analyte binding pair can be nucleic acid to nucleic acid, e.g. DNA/DNA, DNA/RNA, RNA/DNA, RNA/RNA, RNA. The binding element/analyte binding pair can be a polypeptide and a nucleic acid, e.g. polypeptide/DNA and polypeptide/RNA, such as a sequence specific DNA binding protein. The binding element/analyte binding pair can be any nucleic acid, including synthetic DNA/RNA binding ligands (such as polyamides) capable of sequence-specific DNA or RNA recognition. The binding element/analyte binding pair can comprise natural binding compounds such as natural enzymes and antibodies, and synthetic binding compounds. The binding element/analyte binding can comprise aptamers, which are nucleic acid or polypeptide species that have been engineered to have specific binding properties, usually through repeated rounds of in vitro selection or equivalently, SELEX (systematic evolution of ligands by exponential enrichment).

Binding elements and/or analytes as provided herein can be any type of organic or inorganic molecules or compounds, for example, biomolecules. In some cases, the binding elements and/or analytes comprise proteins, peptides, antibodies, antigen-binding antibody fragments, polysaccharides, enzymes, aptamers, nucleic acids, antigens, cells, or tissues. In some cases, the protein may be derived from cell or tissue lysate, body fluid, or other sample source, such as in the case of reverse phase protein array analysis. For example, the binding elements and/or analytes can be antigens for detecting antibodies in a sample. Non-limiting examples of antibodies include, total human IgG, IgM, IgA and IgE; anti-HLA (human leukocyte antigen) antibodies; anti-dsDNA antibodies; anti-Smith antibodies; antibodies diagnostic of Systemic Lupus Erythematosus (SLE), Toxoplasmosis, Rubella, Rabies, Malaria, lyme disease, African Trypanosomiasis, cholera, cryptosporidiosis, dengue, influenza, Japanese Encephalitis, Leishmaniasis, measles, meningitis, onchocerciasis, pneumonia, tuberculosis, typhoid, or yellow fever; antibodies specific for cytomegalovirus (CMV), Toxoplasma gondii, Rubella virus, Herpes simplex virus 1 and 2 (HSV-1/2), anti-Hemoglobin Alpha (HBA), Hepatitis B virus (HBV), Hepatitis C virus (HCV), Hepatitis D Virus (HDV), human immunodeficiency virus (HIV); Human papillomavirus (HPV), Ebola virus, rotavirus, human leukocyte antigens, Thyroid Stimulating Hormone Receptor (TSHR), thyroperoxidate, Thyroglobulin, tissue transglutaminase (tTG), endomysium, deamidated gliadin peptide; antibodies specific for tumor-associated antigens selected from p53, NY-ESO-1, MAGE A4, HuD, CAGE, GBU4-5, and SOX2, or combinations thereof. Multiple diagnostic antibodies may be assessed in a single assay, such as antibodies for Toxoplasma gondii, Rubella, cytomegalovirus (CMV), and herpes simplex virus (HSV), as in the case of a TORCH assay. TORCH infections are a group of congenitally acquired infections that cause significant morbidity and mortality in neonates. These infections are acquired by the mother and passed either transplacentally or during the birth process. While each infection is distinct, there are many similarities in how these infections present. It is important to consider TORCH infections whenever a neonate presents with intrauterine growth restriction (IUGR), microcephaly, intracranial calcifications, conjunctivitis, hearing loss, rash, hepatosplenomegaly, or thrombocytopenia. Although the five classic infections are mentioned above, other categories of infections may also assessed (e.g. in the same binding reaction), such as human immunodeficiency virus (HIV), varicella zoster virus (VZV), Herpes, Syphilis, parvovirus B19, enteroviruses, and others. Examples of antibodies of a TORCH assay may include, but is not limited to Toxoplasma gondii Antibody, Immunoglobulin G (IgG); Rubella Antibody, IgG; Herpes Simplex Virus Type 1 and/or 2 antibodies, IgG; Cytomegalovirus Antibody, IgG; Toxoplasma gondii IgM Antibody, Immunoglobulin M (IgM); Rubella Antibody, IgM; Herpes Simplex Virus Type 1 and/or 2 Antibodies, IgM; and Cytomegalovirus Antibody, IgM. In a typical TORCH assay, the binding element is an antigen (e.g. one or more Toxoplasmosis gondii antigens, one or more Rubella antigens, one or more CMV antigens, and one or more HSV antigens) and the analyte is an antibody directed against one or more of the antigens.

In general, an antibody diagnostic of a given condition is one that binds a molecule associated with that condition. In the case of infection, the antibody may be one that binds a protein of the infectious agent, such as a viral capsid protein or a bacterial cell surface protein. In some cases, the antigen is an infectious agent or component thereof. Where the disease is an autoimmune disorder, the antibody may be an autoantibody, and the antigen is a human protein or portion thereof.

In some cases, analytes can comprise a biomarker. The biomarker may be associated with a biological state or condition of an organism, such as a subject. Examples of such biological state or condition include, without limitation, a disease, a disorder, a non-disease condition, a healthy condition, or therapeutic responses to different drug treatments and other therapies. For example, the analyte can be an inflammatory cytokine, a biomarker for cardiovascular disease, a biomarker for infectious disease, a biomarker for inflammatory disease (e.g. inflammatory bowel disease), or a biomarker for cancer. Non-limiting examples of biomarkers include glycated proteins, glycated hemoglobin (HbA1c), HbA-Glyc, HbA-SNO, glycated albumin (GA), glucose (e.g., fasting plasma glucose), human serum albumin (HSA), HSA-Cys, HSA-Glyc, apolipoprotein A-I (apoA-I), apoA-I MetO, GA, glycated apolipoprotein A-1 (GapoA-I), Alpha-fetoprotein (AFP), philadelphia chromosome (BCR-ABL), breast cancer type 1 susceptibility protein (BRCA1), breast cancer type 2 susceptibility protein (BRCA2), v-Raf murine sarcoma viral oncogene homolog B (BRAF V600E), carcinoma antigen 125 (CA-125), carbohydrate antigen 19-9 (CA19.9), Zn-α2 glycoprotein (ZAG), carcinoembryonic antigen (CEA), epidermal growth factor receptor (EGFR), receptor tyrosine-protein kinase erbB-2 (HER-2), mast/stem cell growth factor receptor (KIT), prostate-specific antigen (PSA), S-100 proteins (S100), total tau (T-tau), hyperphosphorylated tau (P-tau), 42 amino acid isoform of amyloid β (Aβ42), cytokines (e.g., interleukin (IL)-1, IL-6, IL-8, Il-10, IL-1β, IL-1Ra, TNF-α monocyte chemoattractant protein-1 (MCP-1) etc.), soluble CD40 ligand, serum amyloid A (SAA), selectins (e.g., E-selectin, P-selectin), myeloperoxidase (MPO), matrix metalloproteinases (MMPs), cellular adhesion molecules (e.g., intercellular adhesion molecule 1 (ICAM-1), vascular adhesion molecule 1 (VCAM-1)), placental growth factor (PlGF), A2 phospholipases, high-sensitivity C-reactive protein (hs-CRP), metalloproteinases (MMP-9, MMP-11), pregnancy-associated plasma protein A (PAPP-A), cathepsin S, chemotactic molecules (MCP-1, CCR1, CCR2), myeloperoxidase, neopterin, growth differentiation factor-15, placental growth factor, markers of fibrosis (e.g., galectin-3), fetuin-A, vascular calcification (osteoprogenterin), myeloid-related proteins 8/14 (MRP8/14), pentraxin 3 (PTX3), osteoprotegerin, von Willebrand factor (vWF), tissue factor (TF), soluble CD40 ligand (sCD40L), prothrombin fragment 1.2 (F1.2), thrombus precursor protein (TpP), D dimer, Lp-PLA2 mass, oxidized amino acids, oxidised apolipoprotein A1 (apoA1), asymmetric dimethylarginine (ADMA), secretory phospholipase, high-sensitivity cardiac troponin, malondialdehyde-modified low-density lipoprotein, heart-type Fatty Acid-Binding Protein (H-FABP), B-type natriuretic peptide (BNP), N-terminal pro b-type natriuretic peptide (NT-proBNP), copeptin, mid-region pro-adrenomedullin, urocortin-1, arginine vasopressin (AVP), endothelin-1, galectin-3, ST-2, cystatin-C, neutrophil gelatinase-associated lipocalin (NGAL), KIM, adiponectin, leptin, resistin, c-peptide, phospholipid fatty acids (EPA and DHA), apolipoprotein E (ApoE), Cholesteryl ester transfer protein (CETP), S100 calcium binding protein B (S100 Beta), Neuron-specific enolase (NSE), and fractions, derivatives or combinations thereof. In some embodiments, the biomarker is a biomarker of an inflammatory bowel disease, non-limiting examples of which include C-reactive protein (CRP), α₁ acid glycoprotein (orosomucoid), perinuclear antineutrophil cytoplasmic antibodies (pANCA), anti-Saccharomyces cerevisiae antibodies (ASCA), fecal calprotectin, nucleotide-binding oligomerization domain-containing protein 2 (NOD2), anti-Outer Membrane Protein C (anti-OmpC), pancreatic autoantibodies (PAB), anti-laminaribioside carbohydrate antibodies (ALCA), anti-chitobioside carbohydrate antibodies (ACCA), anti-mannobioside carbohydrate antibodies (AMCA), anti-laminarin (anti-L), anti-Cbirl flagellin, anti-I2 antibody, anti-chitin (anti-C) antibody, antibodies against goblet cells (GAB), and antibodies to bacterial flagellin CBir1 (anti-CBir1). Panels of biomarkers may be assayed in a single reaction, and may include biomarkers for a plurality of conditions and/or a plurality of biomarkers for each of one or more conditions.

In some cases, the biomarker is a biomarker associated with cardio vascular disease (CVD). The term "cardiovascular disease" (CVD) generally refers to a number of diseases that affect the heart and circulatory system, including aneurysms; angina; arrhythmia; atherosclerosis; cardiomyopathies; cerebrovascular accident (stroke); cerebrovascular disease; congenital heart disease; congestive heart failure; coronary heart disease (CHD), also referred to as coronary artery disease (CAD), ischemic heart disease or atherosclerotic heart disease; dilated cardiomyopathy; diastolic dysfunction; endocarditis; heart failure; hypertension (high blood pressure); hypertrophic cardiomyopathy; mitral valve prolapse; myocardial infarction (heart attack); myocarditis; peripheral vascular disease; rheumatic heart disease; valve disease; and venous thromboembolism. As used herein, the term "cardiovascular disease" also encompasses reference to ischemia; arterial damage (damage to the endothelial lineage) due to physical damage (endartiectomie, balloon angioplasty) or as a result of chronic damage (including atherosclerosis); myocardial damage (myocardial necrosis); and myonecrosis. In general, any physiological or pathophysiological condition that elicits a neoangiogenic response is encompassed by the term "cardiovascular disease" as used herein. A biomarker may be considered associated with CVD when present at a higher level as compared to the level of the same biomarker in a subject or population of subjects not suffering from CVD. Examples of biomarkers associated with cardiovascular disease include, but are not limited to, troponin, troponin I (cTnI), creatinin kinase MB subfraction, ADORA1, ADORA2A, ADORA2B, ADORA3, AGTRL1 (APLNR), AMPH, APLN, CCBE1, CDC42, CGNL1, CREBBP, CRIP1, CRIP2, CRIP3, CYB5B, DLL4, DUSP5, EEA1, egr-1, ELK1, ELK3, ELK4 (SAP1), EP300, ERG1 (KCNH2), ETS1, ETS2, EXOC3L, FGD1, FGD2, FGD3, FGD4, FGD5, FLT1, FST, GATA6, GRRP1, HO-1 (HMOX1), HO-2 (HMOX2), IFNG, ILIA, IL1B, LAMA4, Lamb1-1, LGMN, MMP3, Nos2, PAI1, PHD1, PLVAP, RAB5a, RIN3, ROCK2, SOX18, SOX7, SRF, STAB1, STAB2, STUB1, TFEC, THBS1, THBS2, THBS3, THBS4, THBS5, THSD1, TNFAIP8, and XLKD1 (LYVE1). In some embodiments, the marker is one or more of (e.g. 2, 3, 4, 5, 10, 15, 20, 25, 30 or all) genes selected from the group consisting of ADORA2A, AGTRL1 (APLNR), APLN, CCBE1, CGNL1, CRIP2, CYB5B, DLL4, DUSP5, ELK3, ERG1 (KCNH2), ETS1, ETS2, EXOC3L, FGD5, GRRP1, HO-1 (HMOX1), HO-2 (HMOX2), LAMA4, Lamb1-1, LGMN, PLVAP, RIN3, ROCK2, SOX7, SOX18, STAB1, STAB2, STUB1, TFEC, THSD1, TNFAIP8, and XLKD1 (LYVE1). In some embodiments, the CVD biomarker is an auto-antibody, such as an antibody directed to Annexin A5, SDHA, ATP1A3, titin, myosin, ADBRK, EDNRA, EDNRB, AGTR1, CHRM2, HSPD. Biomarkers may be detected at the polynucleotide level (e.g. genomic level, in cell-free DNA, or mRNA expression level) or at the protein level. Methods employing such biomarkers may comprise one or more steps selected from obtaining a sample from a subject, detecting an analyte in a sample from a subject, diagnosing a subject as having or being at risk for CVD, and taking medical action on the basis analyte detection.

In some cases, the biomarker is a biomarker associated with cancer. In general, the term "cancer" refers broadly to any neoplastic disease (whether invasive or metastatic) characterized by abnormal and uncontrolled cell division causing malignant growth or tumor (e.g., unregulated cell growth). Non-limiting examples of cancer include Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Mtillerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sézary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenström's macroglobulinemia, Warthin's tumor, and Wilms' tumor. A marker can be considered associated with a particular cancer when it is present in a subject having such cancer at a higher level than in a subject not having such cancer, or expressed at a higher level in a cancer tissue than in normal tissue of the same type. Examples of specific associations include, but are not limited to, acute lymphoblastic leukemia (etv6, am11, cyclophilin b), B cell lymphoma (Ig-idiotype), glioma (E-cadherin, α-catenin, β-catenin, γ-catenin, p120ctn), bladder cancer (p21ras), biliary cancer (p21ras), breast cancer (MUC family, HER2/neu, c-erbB-2), cervical carcinoma (p53, p21ras), colon carcinoma (p21ras, HER2/neu, c-erbB-2, MUC family), colorectal cancer (Colorectal associated antigen (CRC)-CO17-1A/GA733, APC), choriocarcinoma (CEA), epithelial cell cancer (cyclophilin b), gastric cancer (HER2/neu, c-erbB-2, ga733 glycoprotein), hepatocellular cancer (a-fetoprotein), Hodgkins lymphoma (Imp-1, EBNA-1), lung cancer (CEA, MAGE-3, NY-ESO-1), lymphoid cell-derived leukemia (cyclophilin b), melanoma (p5 protein, gp75, oncofetal antigen, GM2 and GD2 gangliosides, Melan-A/MART-1, cdc27, MAGE-3, p21ras, gp100^{Pmel117}), myeloma (MUC family, p21ras), non-small cell lung carcinoma (HER2/neu, c-erbB-2), nasopharyngeal cancer (Imp-1, EBNA-1), ovarian cancer (MUC family, HER2/neu, c-erbB-2), prostate cancer (Prostate Specific Antigen (PSA) and its antigenic epitopes PSA-1, PSA-2, and PSA-3, PSMA, HER2/neu, c-erbB-2, ga733 glycoprotein), renal cancer (HER2/neu, c-erbB-2), squamous cell cancers of the cervix and esophagus (viral products such as human papilloma virus proteins), testicular cancer (NY-ESO-1), and T cell leukemia (HTLV-1 epitopes). Methods employing such cancer biomarkers may comprise one or more steps selected from obtaining a sample from a subject, detecting an analyte in a sample from a subject, diagnosing a subject as having or being at risk for cancer, and taking medical action on the basis analyte detection.

In some cases, the biomarker is a biomarker for an autoimmune disease, such as an auto-antibody. Examples of autoimmune diseases include but are not limited to inflammation, antiphospholipid syndrome, systemic lupus erythematosus, rheumatoid arthritis, autoimmune vasculitis, celiac disease, autoimmune thyroiditis, post-transfusion immunization, maternal-fetal incompatibility, transfusion reactions, immunological deficiency such IgA deficiency, common variable immunodeficiency, drug-induced lupus, diabetes mellitus, Type I diabetes, Type II diabetes, juvenile onset diabetes, juvenile rheumatoid arthritis, psoriatic arthritis, multiple sclerosis, immunodeficiency, allergies, asthma, psoriasis, atopic dermatitis, allergic contact dermatitis, chronic skin diseases, amyotrophic lateral sclerosis, chemotherapy-induced injury, graft-vs-host diseases, bone marrow transplant rejection, Ankylosing spondylitis, atopic eczema, Pemphigus, Behcet's disease, chronic fatigue syndrome fibromyalgia, chemotherapy-induced injury, myasthenia gravis, glomerulonephritis, allergic retinitis, systemic sclerosis, subacute cutaneous lupus erythematosus, cutaneous lupus erythematosus including chilblain lupus erythematosus, Sjögren's syndrome, autoimmune nephritis, autoimmune vasculitis, autoimmune hepatitis, autoimmune carditis, autoimmune encephalitis, autoimmune mediated hematological diseases, lc-SSc (limited cutaneous form of scleroderma), dc-SSc (diffused cutaneous form of scleroderma), autoimmune thyroiditis (AT), Grave's disease (GD), myasthenia gravis, multiple sclerosis (MS), ankylosing spondylitis. transplant rejection, immune aging, rheumatic/autoimmune diseases, mixed connective tissue disease, spondyloarthropathy, psoriasis, psoriatic arthritis, myositis, scleroderma, dermatomyositis, autoimmune vasculitis, mixed connective tissue disease, idiopathic thrombocytopenic purpura, Crohn's disease, human adjuvant disease, osteoarthritis, juvenile chronic arthritis, a spondyloarthropathy, an idiopathic inflammatory myopathy, systemic vasculitis, sarcoidosis, autoimmune hemolytic anemia, autoimmune thrombocytopenia, thyroiditis, immune-mediated renal disease, a demyelinating disease of the central or peripheral nervous system, idiopathic demyelinating polyneuropathy, Guillain-Barre syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease (including Crohn's disease (CD) and ulcerative colitis (UC)), gluten-sensitive enteropathy, Whipple's disease, an autoimmune or immune-mediated skin disease, a bullous skin disease, erythema multiforme, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease, psoriatic arthritis, psoriasis, dermatitis, polymyositis/dermatomyositis, toxic epidermal necrolysis, systemic scleroderma and sclerosis, responses associated with inflammatory bowel disease, Crohn's disease, ulcerative colitis, respiratory distress syndrome, adult respiratory distress syndrome (ARDS), meningitis, encephalitis, uveitis, colitis, glomerulonephritis, allergic conditions, eczema, asthma, conditions involving infiltration of T cells and chronic inflammatory responses, atherosclerosis, autoimmune myocarditis, leukocyte adhesion deficiency, allergic encephalomyelitis, immune responses associated with acute and delayed hypersensitivity mediated by cytokines and T-lymphocytes, tuberculosis, sarcoidosis, granulomatosis including Wegener's granulomatosis, agranulocytosis, vasculitis (including ANCA), aplastic anemia, Diamond Blackfan anemia, immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, central nervous system (CNS) inflammatory disorders, multiple organ injury syndrome, mysathenia gravis, antigen-antibody complex mediated diseases, anti-glomerular basement membrane disease, anti-phospholipid antibody syndrome, allergic neuritis, Bechet disease, Castleman's syndrome, Goodpasture's syndrome, Lambert-Eaton Myasthenic Syndrome, Reynaud's syndrome, Sjögren's syndrome, Stevens-Johnson syndrome, pemphigoid bullous, pemphigus, autoimmune polyendocrinopathies, Reiter's disease, stiff-man syndrome, giant cell arteritis, immune complex nephritis, IgA nephropathy, IgM polyneuropathies or IgM mediated neuropathy, idiopathic thrombocytopenic purpura (ITP), thrombotic throbocytopenic purpura (TTP), autoimmune thrombocytopenia, autoimmune disease of the testis and ovary including autoimmune orchitis and oophoritis, primary hypothyroidism, autoimmune endocrine diseases including autoimmune thyroiditis, chronic thyroiditis (Hashimoto's Thyroiditis), subacute thyroiditis, idiopathic hypothyroidism, Addison's disease, Grave's disease, autoimmune polyglandular syndromes (or polyglandular endocrinopathy syndromes), Sheehan's syndrome, autoimmune hepatitis, lymphoid interstitial pneumonitis (HIV), bronchiolitis obliterans (non-transplant) vs NSIP, Guillain-Barre' Syndrome, large vessel vasculitis (including polymyalgia rheumatica and giant cell (Takayasu's) arteritis), medium vessel vasculitis (including Kawasaki's disease and polyarteritis nodosa), ankylosing spondylitis, Berger's disease (IgA nephropathy), rapidly progressive glomerulonephritis, primary biliary cirrhosis, Celiac sprue (gluten enteropathy), cryoglobulinemia, and amyotrophic lateral sclerosis (ALS). In some cases, the autoimmune disease is SLE, rheumatoid arthritis, or celiac's disease. Examples of biomarkers for autoimmune diseases include those described in US20070141625, US20090226440, US20090263474, US20100075891, US20100104579, US20100105086, US20100131286, US20100144055, US20100151471, US20090176217, US20090202469, US20020119118, US20080213280, US20090023166, US20080221016, US20080194474, US20070224638, US20070135335, US20070128189, US20070122413, US20090130683, US20090110667, and US20090023166.

As provided in the present disclosure, any of the binding elements and/or analytes may be tagged with one or more reporting molecules (or labels). The labels may comprise fluorescent molecules. Binding of the binding elements and the analytes that comprise the fluorescent molecules may produce a fluorescent signal that may be enhanced by a film of the present disclosure. In some cases, the binding elements and/or analytes may be tagged with a primary antibody, which may be bound by a secondary antibody comprising at least one fluorescent molecule. Occurrence of binding events between the binding elements and the analytes may then produce a fluorescent signal that can be enhanced with the presence of the film.

In some cases, the binding element is an antibody, which may be used for capturing, labeling, or otherwise detecting an analyte. An "antibody" is an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact polyclonal or monoclonal antibodies, but also fragments thereof (such as Fab, Fab', F(ab')2, Fv), single chain (ScFv), mutants thereof, fusion proteins comprising an antibody portion (such as domain antibodies), and any other modified configuration of the immunoglobulin molecule that comprises an antigen recognition site. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2. The heavy-chain constant domains that correspond to the different classes of immunoglobulins are called alpha, delta, epsilon, gamma, and mu, respectively.

The antibody may be a monoclonal antibody. As used herein, "monoclonal antibody" refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies can be made by the hybridoma method first described by Kohler and Milstein, 1975, Nature, 256:495, or may be made by recombinant DNA methods such as described in U.S. Pat. No. 4,816,567. The monoclonal antibodies may also be isolated from phage libraries generated using the techniques described in McCafferty et al., 1990, Nature, 348:552-554, for example.

### Film fabrication

In one aspect, the disclosure provides methods for making a film described herein. In one embodiments of the disclosure, the method comprises the steps of: (a) adsorbing nanoparticle seeds, such as gold nanoparticle seeds, on a substrate, or growing nanoparticle seeds in a solution or vapor phase on a substrate; and (b) growing nanostructures, such as silver nanostructures, around the nanoparticle seeds. The nanoparticles seeds and the nanostructures grown around the nanoparticle seeds can be made of various types of materials, for example, small molecules, chemical compounds, polymers or metals. The substrate may or may not be treated or modified prior to the seeding process. An example method for preparing the film of the present disclosure may comprise: (a) applying to a substrate a first solution containing metal ions; (b) precipitating the metal ions from the solution onto the substrate using a basic solution; (c) reducing the metal ions precipitated onto the substrate in step (b) to produce seed particles on the substrate; and (d) adding a second solution comprising metal ions to the seed particles from step (c) to grow isolated areas in a film. As discussed elsewhere herein, the film can be continuous, quasi-continuous or discontinuous. Once the film is prepared, in some cases, an array of biological or chemical molecules used as capture agents or binding elements that can specifically bind to one or more analytes may be applied to the film. The array of molecules may be disposed as different molecular species at discrete locations on the film and coupled to the film, whereby fluorescent signals produced by the capturing events between the binding elements and the analytes can be enhanced by the film. As will be appreciated, the first and the second solutions of metal ions may comprise the same type of metal, or different types of metals to form a metallic composite. For example, in some cases, the first solution comprises a plurality of Au ions and therefore Au(0) seed particles are produced on a substrate. The second solution provided may comprise Au or Ag ions, which may grow Au or Ag nanostructures around Au(0) seeds. Depending on the inter-connectivity of produced nanostructures, a continuous, discontinuous, or quasi-continuous Ag/Au or Au/Au film may be fabricated.

Height or thickness of a film may vary. In some cases, the height of the film is less than or equal to about 5,000 nm, 4,000 nm, 3,000 nm, 2,000 nm, 1,000 nm, 900 nm, 800 nm, 700 nm, 600 nm, 500 nm, 400 nm, 300 nm, 200 nm, 100 nm, 90 nm, 80 nm, 70 nm, 60 nm, 50 nm, 40 nm, 30 nm, 20 nm, 10 nm, 9 nm, 8 nm, 7 nm, 6 nm, 5 nm, 4 nm, 3 nm, 2 nm, 1 nm, 0.5 nm, 0.1 nm, 0.05 nm, 0.01 nm, 0.005 nm, or 0.001 nm. In some cases, the height of the film may be greater than or equal to about 0.0001 nm, 0.00025 nm, 0.0005 nm, 0.00075 nm, 0.001 nm, 0.0025 nm, 0.005 nm, 0.0075 nm, 0.01 nm, 0.025 nm, 0.05 nm, 0.075 nm, 0.1 nm, 0.25 nm, 0.5 nm, 0.75 nm, 1 nm, 2 nm, 3 nm, 4 nm, 5 nm, 6 nm, 7 nm, 8 nm, 9 nm, 10 nm, 15 nm, 20 nm, 25 nm, 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 200 nm, 300 nm, 400 nm, 500 nm, 600 nm, 700 nm, 800 nm, 900 nm, 1,000 nm, 1,500 nm, 2,000 nm, 2,500 nm, or 3,000 nm. In some cases, the height of the film may be between any of the two values described herein, for example, between about 5 nm to about 500 nm.

With a film of the present disclosure, fluorescent signals produced within a certain distance of the surface of the film may be enhanced, with enhancement factor varying as a function of a number of factors including such distance. The fluorescent signals may be in the range of about 400 nm to about 2100 nm. Other non-limiting examples of factors that may influence the enhancement factor of a fluorescent signal may include film characteristics and/or features (film thickness, gap size, size of raised nanostructures, type of substrate, shape of substrate, roughness of film surface, roughness of substrate surface), type of fluorescent molecules, light source, or combinations thereof. For example, given a certain film, the fluorescent signal may be enhanced for fluorophores within 1,000 nm of the surface of the film. In some examples, the fluorophore is a near-infra-red fluorophore having an emission of about 700 nm to about 800 nm, and the intensity of the fluorescent signal is enhanced by at least 30-fold. In some examples, the fluorophore is a visible dye having an emission of about 400 nm to about 700 nm, and the intensity of the fluorescent signal is enhanced by at least about 3-fold.

In cases where seed particles are prepared by precipitation out of solution of metal ions, the seeding density may be varied by tuning the initial concentration and/or pH values of the solution. The seeding density may in turn determine the film density and morphology (e.g., gap size, inter-particle spacing, nanoparticle sizes etc.). In some cases, a high seeding density may be required. In some cases, a low seeding density may be preferred. In some cases, the seeding density may be less than or equal to about 1 x 10⁹ seeds/mm², 5 x 10⁸ seeds/mm², 1 x 10⁸ seeds/mm², 5 x 10⁷ seeds/mm², 2.5 x 10⁷ seeds/mm², 1 x 10⁷ seeds/mm², 5 x 10⁶ seeds/mm², 1 x 10⁶ seeds/mm², 5 x 10⁵ seeds/mm², 1x 10⁵ seeds/mm², 5 x 10⁴ seeds/mm² or 1 x 10⁴ seeds/mm². In some cases, the seeding density may be greater than or equal to about 1 x 10⁴ seeds/mm², 5 x 10⁴ seeds/mm², 1 x 10⁵ seeds/mm², 5 x 10⁵ seeds/mm², 1 x 10⁶ seeds/mm², 5 x 10⁶ seeds/mm², 1 x 10⁷ seeds/mm², 5 x 10⁷ seeds/mm², 1 x 10⁸ seeds/mm², 5 x 10⁸ seeds/mm², 1 x 10⁹ seeds/mm² or 5 x 10⁹ seeds/mm². In some cases, the seeding density may be between any of the two values described herein, for example, about 4.3 x 10⁷ seeds/mm².

In some embodiments of the disclosure, the methods and materials employ solution phase growth of plasmonic discontinuous silver-on-gold (dAg/Au) and continuous silver-on-gold (cAg/Au) films in a method that begins with rapid, *in situ* "seeding" of gold nanoparticles by deposition/precipitation of Au³⁺ ions onto unmodified surfaces, followed by solution-phase reduction of the ions to Au⁰. For Ag/Au structures, subsequent to the reduction step, the gold seeds are grown into a film by the glucose reduction of Ag¹⁺ and the resulting films with different degrees of growth are referred to as continuous silver-on-gold (cAg/Au) or discontinuous silver-on-gold (dAg/Au) films. In some embodiments of the disclosure, this involves a three step process in the preparation of the present nanoscopic cAg/Au and dAg/Au films:
(1) seeding of gold onto a substrate by precipitation out of solution of Au³⁺ ions. The ions are precipitated from HAuCl₄ by raising its pH with a nitrogenous base, such as with NH₄OH, urea, etc;
(2) reducing the ions precipitated in step (1) to Au⁰ clusters on the substrate by a reducing agent such as Hydrazine, NaBH₄, heat, H₂, or photo-reduction ; and
(3) growing seeds from step (2) by selectively adding silver to the initial seeds by reduction of an Ag¹⁺ halide in a second solution to form nanoplates and raised structures. This can be done by a reducing agent such as photo-reduction, D-glucose, or ultrasound treatment in a hydrogen enriched atmosphere. Typically, the silver in step (3) only attaches to the previously deposited seeds, leading to the so-called "cAg/Au" and "dAg/Au" construction.

The initial seeding (precipitation) step can be carried out on a variety of substrates by immersing the substrate in the ionic gold solution. The substrate does not need to be but can be pretreated in any way to increase gold adhesion. The ionic concentration of the gold salt is selected to control the size and spacing of the "seeds." Without wishing to be bound by theory, it is believed that the final size of and distance between nanoplates affects the fluorescent enhancement properties of the substrate and can be optimized to maximize fluorescence enhancement in the visible and near-infrared region. As described below, near infrared fluorescence from an infrared fluorophore (IRDye800) was increased 10-200 fold by controlling the cAg/Au nano-nanoplates size to be on the order of hundreds of nanometers spaced at several to tens of nanometer gaps. As described below, visible fluorescence from a visible fluorophore (Cy5) and a near-infrared fluorophore (IRDye800) were increased between 2-100-fold as compared with bare glass substrates by controlling the cAg/Au raised nanostructure size to have average feature width between 5 nm to 100 nm and gap width of 1 to 20 nm.

As the uniformity and morphology of the film as described herein can be easily tuned, assays or methods utilizing the film to capture or detect fluorescent signals can be highly multiplexed. Different types of capture agents (or binding elements) can be deposited on different locations of the film, wherein each location may have different morphology or properties (e.g., size, shape and density of nanostructures, gap size, roughness of the surface of the film etc.). It can enable multiplexed detection of up to hundreds or thousands of analytes (e.g., cytokines or other proteins) in an array with substantially lower limit of detection (e.g., down to about 0.01 pg/mL (∼1-10 fM) minimum detectable concentration), with high sensitivity, specificity and signal-to-noise ratio. Different fluorescent molecules with non-overlapping emission wavelengths can be used in the same assay to label different classes of analytes (e.g., proteins or antibodies) to achieve multi-color differentiation of subtypes of analytes such as IgG, IgM, IgA in the same assay. For example, the film can be utilized to build multi-color microarrays capable of measuring different sub-types of antibodies with low and high abundances in human serum, with the maximally enhanced fluorophore for reporting the least abundant molecule.

As provided herein, the ratio of the signal to the noise (e.g., ratio of their amplitudes) can be any suitably high value (i.e., suitably high to achieve a certain accuracy). For example, the signal to noise ratio may be at least about 2 to 1 , about 3 to 1 , about 4 to 1 , about 5 to 1 , about 6 to 1 , about 7 to 1, about 8 to 1, about 9 to 1, about 10 to 1, about 100 to 1, about 1,000 to 1, about 10,000 to one, or more.

Reduction of ions contained in the first and/or the second solutions may be achieved by various methods, for example, via thermal- or photo-induced reduction, or with the aid of chemical reagents such as reducing agents. Non-limiting examples of reducing agents may include Ascorbic acid, Hydrazine, Hydroxylamine, Ammonium or sodium borohydrate, Formic acid, D-glucose, a hydrogen gas atmosphere, Lithium aluminum hydride (LiAlH₄), Nascent (atomic) hydrogen, Sodium amalgam, Diborane, Sodium borohydride (NaBH₄), Compounds containing the Sn²⁺ ion, such as tin(II) chloride, Sulfite compounds, Hydrazine (Wolff-Kishner reduction), Zinc-mercury amalgam (Zn(Hg)) (Clemmensen reduction), Diisobutylaluminum hydride (DIBAL-H), Lindlar catalyst, Oxalic acid (C₂H₂O₄), Phosphites, hypophosphites, and phosphorous acid, Dithiothreitol (DTT) - used in biochemistry labs to avoid S-S bonds, Compounds containing the Fe²⁺ ion, such as iron(II) sulfate, Carbon monoxide(CO), Carbon (C), Tris(2-carboxyethyl)phosphine HCl (TCEP), or combinations thereof.

### Applications

Systems and compositions of the present disclosure can be applied for a wide context of applications. Any methods or techniques that utilize fluorescence detection may fall into the scope of the applications as provided herein. Examples of applications include, but are not limited to, microarrays of proteins, nucleic acids, and antibodies for detection via direct and indirect fluorescence antibody techniques with or without multiplexing, gene sequencing, in vitro diagnostics including fluorescence in situ hybridization (FISH), immunohistochemical staining (IHC), exosome capture for IHC staining and FISH, cell-free DNA capture and quantitation assay, chemical imaging (e.g., Mid-infrared chemical imaging, Near-infrared chemical imaging, Raman chemical imaging, Fluorescence Imaging (Ultraviolet, visible and near infrared regions)), Fluorescence Intensity Decay Shape Microscopy (FIDSAM), Fluorescence anisotropy, Fluorescence correlation spectroscopy (FCS), Fluorescence image-guided surgery (FIGS), Fluorescence Loss in Photobleaching (FLIP), Lattice light-sheet microscopy, immunofluorescence assay (IFA), single molecule wide field imaging, super-resolution imaging techniques such as Stimulated emission depletion (STED) and single molecular localization (PALM/STORM), cellular and tissue imaging by wide field, confocal scanners and laser scanning cytometry instruments, Enzyme-Linked Immunospot (ELISPOT), Fluorescence (Forster) Resonance Energy Transfer (FRET) based imaging, Fluorescence Recovery after Photobleaching (FRAP) based imaging, imaging substrates for circulating tumor cells (CTC), imaging substrates for single molecule and single nanoparticle imaging, and for fluorescence-based microarrays of antigens, antibodies, peptides, DNA, RNA, aptamers, tissues, cells and reverse phase microarrays, or combinations thereof.

Some aspects of the present disclosure provide methods of detecting one or more analytes. The methods may comprise: (a) providing a film as described herein; (b) applying to the film an analyte and a label for the analyte, wherein the label may comprise a fluorescent molecule (e.g., a fluorophore) and the analyte may or may not be bound to the label; and (c) detecting the analyte by detecting a fluorescent signal of the fluorescent molecule, wherein the intensity of the fluorescent signal can be enhanced relative to the fluorescent signal in the absence of the film. Various methods and techniques may be used to detect the fluorescent signals, e.g., Fluorescence spectroscopy (or fluorometry, spectrofluorometry), Fluorescence microscopes, Fluorescence scanners and Flow cytometers. In some examples, the detection of fluorescent signals may comprise imaging by microscopy. In general, methods of detecting one or more analytes may further comprise diagnosing a subject as having a condition associated with the presence, absence, or level of the one or more analytes. For example, detection of a biomarker for the presence of an infectious agent in a sample from a subject may be followed by diagnosing the subject as being infected with or a carrier of the infectious agent. Likewise, detection of one or more cancer-related biomarkers may be followed by a diagnosis of cancer, and so forth, depending on the one or more biomarkers assayed. Multiple examples of such biomarkers are provided herein. In some cases, a method may further comprise taking medical action on the basis of detecting one or more analytes and/or a resulting diagnosis. Medical action can include therapeutic intervention, and further testing. Detection can comprise detecting a fluorescent signal from a complex comprising a binding element, an analyte bound to the binding element, and a fluorescent label, all of which may be complexed to the film. The film, binding element, analyte, and fluorescent label can be any of those described herein.

Methods may utilize any of the films described herein. As an example, a film that can be utilized in the methods may have one or more of characteristics including, e.g., (a) the gaps having widths between about 5 nm to about 50 nm, and lengths between about 5 nm and about 200 nm; (b) the nanostructures having an average width and length between about 50 nm to about 500 nm; (c) the film having a nanoplate size of between about 1000 nm² to about 250,000 nm²; (d) the height of the film being between about 5 nm and about 500 nm; (e) the film comprising irregular features and a heterogenous structure; (f) the film imparting a plasmon from about 400 nm to about 2100 nm; (g) the substrate comprising a flat surface, a curved surface, a spherical surface, or a three-dimensional porous membrane; and (h) the substrate being a bead. The film may comprise silver-on-gold nanoparticles.

In some cases, the methods can further comprise determining the concentration, identity (e.g., a phenotype of a cell, or sample source), and/or location of the analyte, or other characteristic of the sample or sample source based upon the detected fluorescent signal. Examples of characteristics include identity of a sample source (e.g. an individual subject or location), presence or absence of contamination, presence or absence of disease or condition, type of protein, nucleotide sequence, or other identifying characteristic. In some cases, the methods comprise identifying a disease or condition of a sample, or identifying a sample source (e.g. a subject). Examples of these are provided above, and include diseases or conditions are a cardiovascular disease or condition, a kidney-associated disease or condition, a prenatal or pregnancy-related disease or condition, a neurological or neuropsychiatric disease or condition, an autoimmune or immune-related disease or condition, a cancer, an infectious disease or condition, a pediatric disease, disorder, or condition, a mitochondrial disorder, a respiratory-gastrointestinal tract disease or condition, a reproductive disease or condition, an ophthalmic disease or condition, a musculo-skeletal disease or condition, or a dermal disease or condition. Other diseases may be identified, such as by identifying the presence of one or more biomarkers or a degree of match with a biosignature associated with the condition. Examples of biomarkers are provided herein.

Various arrangements for the detection of an analyte via fluorescence are available. For example, a sample comprising an analyte may be applied directly to the surface of the film, such as by adding a sample liquid to a well having an inner surface coated with the film. A sample may be directly contacted to the film after first contacting the sample to a sample surface, such as a slide, which can result in sandwiching the analyte between the sample surface and the film. In yet a further example, a sample containing an analyte is contacted with a sample substrate having a first surface in contact with the sample (such as the inner surface of a well, the top surface of a slide, or a channel within a microfluidic device) and a second surface that is not in contact with the sample (such as the bottom surface of a well or slide), and detection of a fluorescent signal associated with the analyte comprises bringing the second surface in proximity with the film (e.g. contact with, or bringing within 1000 nm). The analyte may be labeled with a fluorescent label at any point preceding detection of the analyte in proximity with the film. When the film is on the surface of a bead, the film is brought in proximity to the analyte to be detected by contacting the sample with the beads. Fluorescence may then be detected, such as in the sample container (e.g. as in a well of a multi-well plate). In some cases, film-coated beads that have been contacted with a sample are analyzed by flow cytometry, fluorescence imaging microscopes, or scanners.

In some cases, an analyte may be on a surface. The analyte may be directly or indirectly attached to the surface. The analyte may be bound to the surface via a binding element. The binding elements may be the same or different, and may be any of a variety of binding elements, examples of which are provided herein. The microarray can comprise a DNA microarray, a RNA microarray, a miRNA microarray, a peptide microarray, a protein microarray, an antibody microarray, or any types of microarray of biological or chemical molecules. In cases where the analyte is bound to the surface, a film of the present disclosure may be applied to the analyte on the surface. As discussed above and elsewhere herein, the film may comprise a plurality of binding elements or capture agents (e.g., an array of binding elements). Each of the binding elements may be configured to bind to the same or a different type of analyte. In cases where a non-uniform film which comprises a plurality of heterogeneous and irregular nanostructures is utilized, the film may comprise a plurality of discrete and isolated locations. The plurality of locations may be independently addressable and each of the locations may include a specific type of binding element that is able to bind to a certain analyte. Binding of each pair of binding element and analyte may produce a fluorescent signal that can be enhanced by the film and captured by a detector. Based on the location of the detected signal, the analyte can be determined or identified. In some examples, the binding element is an oligonucleotide (e.g., a primer) conjugated to the film which is applied to a substrate (e.g., a bead), and the analyte is a target polynucleotide that can hybridize to the oligonucleotide via sequence complementary or an amplification product thereof. Once the target polynucleotide successfully hybridizes to the oligonucleotide, an amplification reaction may be initiated to produce a detectable amplified product (or amplicon). Since the film may be fabricated to include a plurality of isolated and independently addressable locations, and each of the locations may contain only a single analyte, the detection may further comprise single molecule analysis, e.g., single molecule imaging and tracking, or single nanoparticle tracking and imaging.

In some cases, the array of binding elements is a protein array. Protein microarrays can be prepared in a number of ways, such as by spotting the desired proteins onto the film or onto a sample substrate. Exemplary methods for preparing protein arrays are described e.g. in US 2003/0013130, US US 2003/0108726, and US 2009/0088329. In some cases, the array of binding elements is a polynucleotide array, such as in an array of oligonucleotide probes on the film or a sample substrate. Exemplary methods for fabricating polynucleotide arrays include the use of fine-pointed pins onto glass slides, photolithography using pre-made masks, photolithography using dynamic micromirror devices, ink-jet printing, or electrochemistry. Exemplary methods are described e.g. in Fodor et al., 1991, Science 251:767-773; Pease et al., 1994, Proc. Natl. Acad. Sci. U.S.A. 91:5022-5026; Lockhart et al., 1996, Nature Biotechnology 14:1675; U.S. Pat. Nos. 5,578,832; 5,556,752; and 5,510,270.

Any of a variety of binding assays that utilize fluorescence as a means for detecting an analyte may be enhanced through use of a film according to the presence disclosure. In some embodiments, the assay is a receptor based assay. In general, receptor based assays comprise detecting an interaction between two binding partners, an analyte receptor (also referred to as a binding element) and an analyte. In general, an analyte receptor and an analyte in a given pair of binding partners are distinguished on the basis of which one is known (the analyte receptor), and which is being detected (the analyte). As such, exemplary analyte receptors described herein may be detected as analytes in other embodiments, and exemplary analytes as described herein may be used as analyte receptors for detection of respective binding partners in other embodiments. In some embodiments, the analyte receptor, the analyte, or both comprise a protein. Analyte receptors include, but are not limited to: natural or synthetic proteins, cellular receptor proteins, antibodies, enzymes, polypeptides, polynucleotides (e.g. nucleic acid probes, primers, and aptamers), lipids, small organic or inorganic molecules, antigens (e.g. for antibody detection), metal binding ligands, and any other natural or synthetic molecule having a binding affinity for a target analyte. In some embodiments, the binding affinity of an analyte receptor for an analyte is a K_{D} of less than about 5×10⁻⁶M, 1×10⁻⁶M, 5×10⁻⁷M, 1×10⁻⁷M 5×10⁻⁸M, 1×10⁻⁸M, 5×10⁻⁹M, 1×10⁻⁹M, 5×10⁻¹⁰M, 1×10⁻¹⁰M, 5×10⁻¹¹, 1×10⁻¹¹, or less. A variety of analytes and analyte receptors are available, as well as assays employing the same. See e.g. US8435738. In some cases, where an array comprises a plurality of different binding elements, a corresponding plurality of different analytes may be detected in a single reaction. In general, by increasing the fluorescent signal from a label associated with the presence or absence of an analyte, the sensitivity of detecting for that analyte is increased, such that smaller amounts of analyte may be detected above a background level than is possible in the absence of a film of the present disclosure.

*In vitro* diagnostic imaging may consist of adhering cells and cellular vesicles to a substrate, typically surface-modified glass, and detecting certain cellular membrane molecules using a complementary antibody or antigen conjugated to a fluorescent dye. In this method, autofluorescence from the cell and photobleaching of the dye are both concerns. Endogenous auto-fluorescence of cells is highest in the visible region from 350-500 nm, decreasing out towards the near-infrared. By performing cellular imaging in conjunction with a film of the disclosure, fluorescence signal of selected labels can be amplified (e.g. for fluorophores with peak excitation wavelengths at 680 nm and 800 nm regions) with minimal autofluorescence. By amplifying fluorescence 10-200 fold in this region, lower intensity excitation can be used, reducing photobleaching.

In some embodiments, the detection assay comprises detecting amplification of one or more target polynucleotides. Methods of amplification may include, for example, polymerase chain reaction (PCR), strand displacement amplification (SDA), and nucleic acid sequence based amplification (NASBA), and Rolling Circle Amplification (RCA).The amplification method can be temperature cycling or be isothermal. The amplification method can be exponential or linear. For amplifications with temperature cycling, a temperature cycle may generally correspond to an amplification cycle. Isothermal amplifications can in some cases have amplification cycles, such as denaturing cycles, and in other cases, the isothermal amplification reaction will occur monotonically without any specific amplification cycle. The amplification method may be used to amplify specific regions (i.e., target regions), or nucleotide sequences of a nucleic acid molecule (e.g., DNA, RNA). This region can be, for example, a single gene, a part of a gene, or a non-coding sequence.

As described herein, the film may be coated on a substrate, wherein the substrate is a bead. In one particular implementation of this embodiment, the beads are further coated with polynucleotide probes as binding elements. A plurality of beads may be provided, and each bead may be coated with multiple copies of the same polynucleotide, or with a plurality of different polynucleotides. The probes may comprise sequences that are complementary to specific target sequences, or may comprise random or partially random sequences for detecting a plurality of different target sequences. Detection may be by way of hybridization alone, such as where a target polynucleotide bearing a fluorescent label hybridizes to the bead and fluorescence is detected. Alternatively, detection may comprise additional manipulation steps, such as in an amplification reaction in which the probes bound to the beads are extended along target polynucleotides that are used as templates in an amplification reaction. Amplified products may be detected during amplification and/or after amplification is completed. A variety of labels for the detection of amplification products are available, such as ethidium bromide, SYBR green, SYBR blue, DAPI, acriflavine, fluorcoumanin, ellipticine, daunomycin, chloroquine, distamycin D, chromomycin, propidium iodine, Hoeste, SYBR gold, acridines, proflavine, acridine orange, homidium, mithramycin, ruthenium polypyridyls, anthramycin, and other suitable agents.

Immunoassay detection via ELISA is utilized in both clinical diagnostics and as a life science research tool as a method to quantify nucleic acid, antibody, or protein concentration in a solution. In practice, a capture antibody or antigen is coated on the surface of a glass slide or 96-well plate at a given concentration, in order to bind to the target molecule in solution. After binding, a detection antibody or antigen is incubated and luminescence, either via absorption or fluorescence spectroscopy, is used to quantify the concentration of the target analyte in solution, typically using a calibration curve as reference. ELISA performed in the absence of films of the disclosure is typically limited to < 3 logs of dynamic range, as well as a lower limit of detection and quantification at ∼ 1 nanogram analyte/milliliter solution. Many biological molecules have clinically relevant concentrations below 1 ng/ml and dynamic ranges that span 6 or more log, such as cytokines and proteins indicative of a disease-state at an early stage. By performing the detection step with labels in proximity to a film of the disclosure, the dynamic range and limit of detection can be expanded to detect analytes at and even below such ranges. For example, the dynamic range may span at least 3, 4, 5, 6, 7, or more logs.

In some embodiments, a film as described herein may be used in conjunction with any of a variety of microscopy techniques, examples of which are described herein. The label selected will depend on the target analyte and detection technique. In some case, the detection method is fluorescence in situ hybridization (FISH). In a typical implementation of this technique, a labeled polynucleotide (a FISH probe) complementary to a sequence of interest is annealed to fixed chromosomes preparations, and the presence of the sequence of interest as well as the chromosomal localization is detected by microscopy. FISH can be performed by immobilizing the nucleic acids of interest on a substrate including without limitation glass, silicon, or fiber. FISH may also be used quantitatively (Q-FISH) to detect the presence and length of repetitive sequences such as telomeres. This may be done by quantitating the intensity of emitted fluorescence as measured by microscopy. FISH assays utilizing the subject fluorescent compounds can be performed for detecting a specific segment of a DNA molecule or a chromosome. These features can be used in genetic counseling (e.g., prenatal-screens), medicine, and species identification. The assay may be performed directly on the film, or on a substrate (e.g. a slide) under which the film is placed to enhance fluorescence for detection.

Also provided herein are methods for sequencing a nucleic acid molecule by using the systems and compositions of the present disclosure. In one embodiments of the disclosure, the methods may comprise: (a) providing a film as provided herein (e.g. silver-on-gold nanoparticles or gold-on-gold nanoparticles); (b) hybridizing an oligonucleotide to a target polynucleotide; (c) extending the oligonucleotide with one or more bases complementary to corresponding positions on the target sequence in the direction of extension; and (d) identifying the one or more bases added in step (c) by detecting a fluorescent signal of one or more fluorescent molecules (e.g., fluorophores), wherein intensity of the fluorescent signal is enhanced relative to the fluorescent signal of the fluorescent molecules in the absence of the film. The oligonucleotide can be extended by a polymerase or a ligase. The four bases used for extension may comprise the same or a different type of fluorescent molecules. In some cases, each of the four bases may be associated with a different fluorescent molecule such that incorporation of each type of the bases produces a distinguishable detectable signal. By detecting the fluorescent signal, sequence of the nucleic acid molecule can be determined. In some cases, the film is on a plurality of beads, which may be flowing through or conjugated to a flow cell. Sequencing can be performed by any of a variety of sequencing methods, including next-generation sequencing (NGS) platforms. NGS technology can involve sequencing of clonally amplified DNA templates or single DNA molecules in a massively parallel fashion (e.g. as described in Volkerding et al. Clin Chem 55:641-658 [2009]; Metzker M Nature Rev 11:31-46 [2010]). The next-generation sequencing platform can be a commercially available platform. Commercially available platforms include, e.g., platforms for sequencing-by-synthesis, ion semiconductor sequencing, pyrosequencing, reversible dye terminator sequencing, sequencing by ligation, single-molecule sequencing, sequencing by hybridization, and nanopore sequencing. Platforms for sequencing by synthesis are available from, e.g., Illumina, 454 Life Sciences, Helicos Biosciences, and Qiagen. Illumina platforms can include, e.g., Illumina's Solexa platform, Illumina's Genome Analyzer, and are described in Gudmundsson et al (Nat. Genet. 2009 41:1122-6), Out et al (Hum. Mutat. 2009 30:1703-12) and Turner (Nat. Methods 2009 6:315-6), U.S. Patent Application Pub nos. US20080160580 and US20080286795, U.S. Pat. Nos. 6,306,597, 7,115,400, and 7232656. 454 Life Science platforms include, e.g., the GS Flex and GS Junior, and are described in U.S. Pat. No. 7,323,305. Platforms from Helicos Biosciences include the True Single Molecule Sequencing platform. Platforms for ion seminconductor sequencing include, e.g., the Ion Torrent Personal Genome Machine (PGM) and are described in U.S. Pat. No. 7,948,015. Platforms for pryosequencing include the GS Flex 454 system and are described in U.S. Pat. Nos. 7,211,390; 7,244,559; 7264929. Platforms and methods for sequencing by ligation include, e.g., the SOLiD sequencing platform and are described in U.S. Pat. No. 5,750,341. Platforms for single-molecule sequencing include the SMRT system from Pacific Bioscience and the Helicos True Single Molecule Sequencing platform.

Exosomes in a sample (e.g. a body fluid, a cell, tissue culture media, or samples derived therefrom) can be captured on a surface (e.g. slides or beads) coated with the fluorescence enhancing films through immobilized exosome specific binding elements, such as antibodies (e.g. anti-CD63, -CD81 and -CD9), and further subjected to a fluorescence based tagging assay. In some embodiments, the tagging assay is for identification and quantitation of protein biomarkers or nucleic acids including DNA and RNA, or assessing signaling pathways or disease states of a subject from which the sample is derived.

Exosomes may be isolated from biological samples, which may include, for example, cell culture media, tissue, fluid, or any samples that may contain exosomes. Non-limiting examples of biological samples include peripheral blood, sera, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen (including prostatic fluid), Cowper's fluid or pre-ejaculatory fluid, female ejaculate, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates or other lavage fluids. Biological samples may also include samples from the blastocyl cavity, umbilical cord blood, or maternal circulation which may be of fetal or maternal origin. In some cases, the biological sample is a tissue sample or biopsy, from which exosomes may be obtained. For example, if the sample is a solid sample, cells from the sample can be cultured to induce exosome product. In some cases, the sample is ascites fluid from a subject, e.g., ascites fluid from a human subject with ovarian cancer; cell culture media supernatant from a human primary melanoma cell line; cell culture media supernatant from a human primary colon cancer cell line; or murine macrophage, e.g., murine macrophage infected with tuberculosis.

In some cases, exosomes are cancer exosomes. Cancer exosomes may include exosomes obtained or derived from cancer cells and/or tumor cells (primary or cell culture) such as breast cancer, ovarian cancer, lung cancer, colon cancer, hyperplastic polyp, adenoma, colorectal cancer (such as CRC Dukes B or Dukes C-D), high grade dysplasia, low grade dysplasia, prostatic hyperplasia, prostate cancer, melanoma, pancreatic cancer, brain cancer (such as a glioblastoma), hematological malignancy (such as B-Cell Chronic Lymphocytic Leukemia, B-Cell Lymphoma-DLBCL, B-Cell Lymphoma-DLBCL-germinal center-like, B-Cell Lymphoma-DLBCL-activated B-cell-like, and Burkitt's lymphoma), hepatocellular carcinoma, cervical cancer, endometrial cancer, head and neck cancer, esophageal cancer, gastrointestinal stromal tumor (GIST), renal cell carcinoma (RCC) or gastric cancer. Cancer exosomes may also be derives from a premalignant condition, for example, but not limited to, Barrett's Esophagus.

Cell free polynucleotides (e.g., cell free DNA or cfDNA) from a sample (e.g. a body fluid) can be captured on a surface (e.g. slides or beads) coated with the fluorescence enhancing films through immobilized DNA-specific antibodies (e.g. anti-dsDNA, -ssDNA, and -DNA/RNA complexes) or complementary polynucleotides, and further subjected to a fluorescence based tagging assay for identification and quantitation of cfDNA for assessing the biology or disease states of a subject from which the sample was derived. In general, cell-free polynucleotides are extracellular polynucleotides present in a sample (e.g. a sample from which cells have been removed, a sample that is not subjected to a lysis step, or a sample that is treated to separate cellular polynucleotides from extracellular polynucleotides). For example, cell-free polynucleotides include polynucleotides released into circulation upon death of a cell, and are isolated as cell-free polynucleotides from the plasma fraction of a blood sample.

Cell free polynucleotides may be derived from a variety of sources including human, mammal, non-human mammal, ape, monkey, chimpanzee, reptilian, amphibian, or avian, sources. Further, samples may be extracted from variety of animal fluids containing cell free sequences, including but not limited to blood, serum, plasma, vitreous, sputum, urine, tears, perspiration, saliva, semen, mucosal excretions, mucus, spinal fluid, amniotic fluid, lymph fluid and the like. Cell free polynucleotides may be fetal in origin (via fluid taken from a pregnant subject), or may be derived from tissue of the subject itself. Isolation and extraction of cell free polynucleotides may be performed through collection of bodily fluids using a variety of techniques. In some cases, collection may comprise aspiration of a bodily fluid from a subject using a syringe. In some cases collection may comprise pipetting or direct collection of fluid into a collecting vessel.

After collection of bodily fluid, cell free polynucleotides may be isolated and extracted using a variety of techniques. In some cases, cell free polynucleotides may be isolated, extracted and prepared using commercially available kits such as the Qiagen Qiamp® Circulating Nucleic Acid Kit protocol. In some examples, Qiagen Qubit™ dsDNA HS Assay kit protocol, Agilent™ DNA 1000 kit, or TruSeq™ Sequencing Library Preparation; Low-Throughput (LT) protocol may be used.

Generally, cell free polynucleotides are extracted and isolated by from bodily fluids through a partitioning step in which cell free DNAs, as found in solution, are separated from cells and other non-soluble components of the bodily fluid. Partitioning may include, but is not limited to, techniques such as centrifugation or filtration. In some cases, cells are not partitioned from cell free DNA first, but rather lysed. In this example, the genomic DNA of intact cells is partitioned through selective precipitation. Cell free polynucleotides, including DNA, may remain soluble and may be separated from insoluble genomic DNA and extracted. Generally, after addition of buffers and other wash steps specific to different kits, DNA may be precipitated using isopropanol precipitation. Further clean up steps may be used such as silica based columns to remove contaminants or salts. General steps may be optimized for specific applications. Nonspecific bulk carrier polynucleotides, for example, may be added throughout the reaction to optimize certain aspects of the procedure such as yield.

In some embodiments, one or more, or all, of the steps in a method of detecting a fluorescent signal are automated, such as by use of one or more automated devices. In general, automated devices are devices that are able to operate without human direction-an automated system can perform a function during a period of time after a human has finished taking any action to promote the function, e.g. by entering instructions into a computer, after which the automated device performs one or more steps without further human operation. Software and programs, including code that implements any of the methods disclosed herein, may be stored on some type of data storage media, such as a CD-ROM, DVD-ROM, tape, flash drive, or diskette, or other appropriate computer readable medium, which may be executed by one or more processors, such as may be part of a computer system. Various embodiments of the disclosure can also be implemented exclusively in hardware, or in a combination of software and hardware. For example, in one embodiment of the disclosure, rather than a conventional personal computer, a Programmable Logic Controller (PLC) is used. PLCs are frequently used in a variety of process control applications where the expense of a general purpose computer is unnecessary. PLCs may be configured to execute one or a variety of control programs, and are capable of receiving inputs from a user or another device and/or providing outputs to a user or another device, in a manner similar to that of a personal computer. An automated system can include a liquid handler. Examples of liquid handlers include liquid handlers from Perkin-Elmer, Beckman Coulter, Caliper Life Sciences, Tecan, Eppendorf.

### EXAMPLES

The following examples are given for the purpose of illustrating various embodiments in accordance with the disclosure and are not meant to limit the present invention in any fashion.

### Example 1: Solution phase method of silver-on-gold film preparation

This example describes production of silver-on-gold films. Materials used in this example included glass and quartz slides purchased from Fisher Scientific, which were rinsed with acetone, IPA, and methanol prior to use. Silicon wafers with native oxide layers were purchased from the Center for Integrated Systems at Stanford University. Poly(vinyl chloride) coverslips were purchased from Ted Pella, Inc. Sylgard 184 was purchased from Dow Corning and cured by standard procedures. Chloroauric acid trihydrate, hydroxylamine HCl, sodium borohydride and glucose were purchased from Sigma-Aldrich. Ammonium Hydroxide (30% ammonia) was purchased from Fisher Chemicals. Plasmon resonances of cAg/Au films on glass substrates were measured by UV-vis-NIR absorbance spectroscopy by a Cary 300 spectrophotometer, background-corrected for any glass contribution. Seeded gold substrates were prepared at varying HAuCl₄ concentrations as described below on ∼0.25 cm² SiO₂ substrates. Following reduction, the substrates were dried and directly imaged in tapping mode by a Nanoscope III multimode AFM (Veeco) with Nanoscope 5 software for height analysis. cAg/Au films grown on glass and SiO₂ were imaged via scanning electron microscopy due to high surface roughness not amenable to AFM. Images were acquired on an FEI XL30 Sirion SEM with FEG source at 5 kV acceleration voltage.

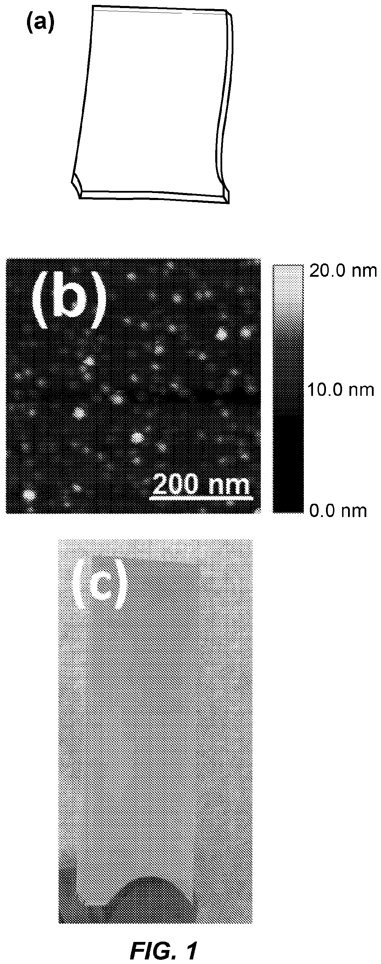

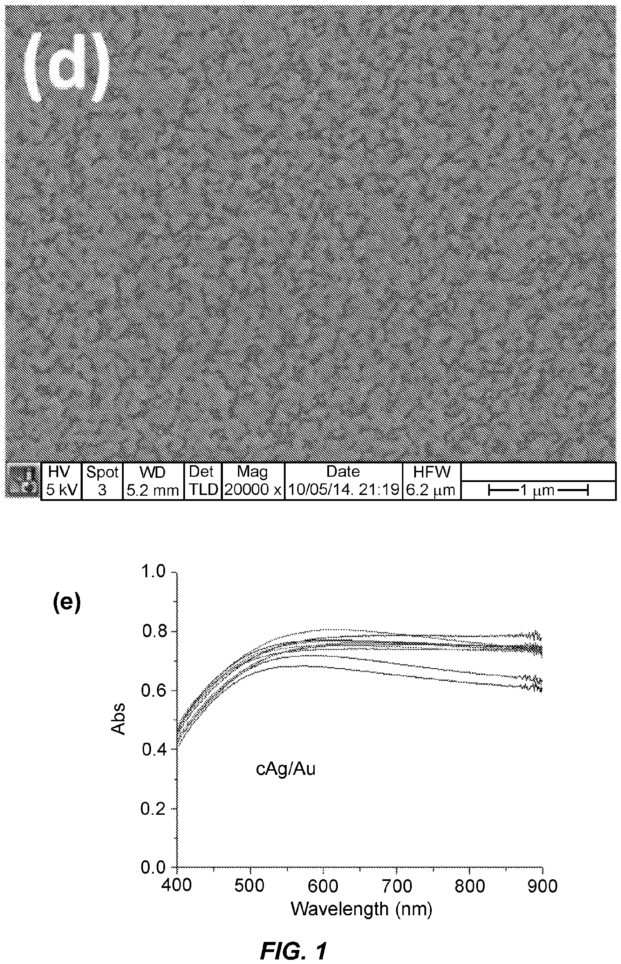

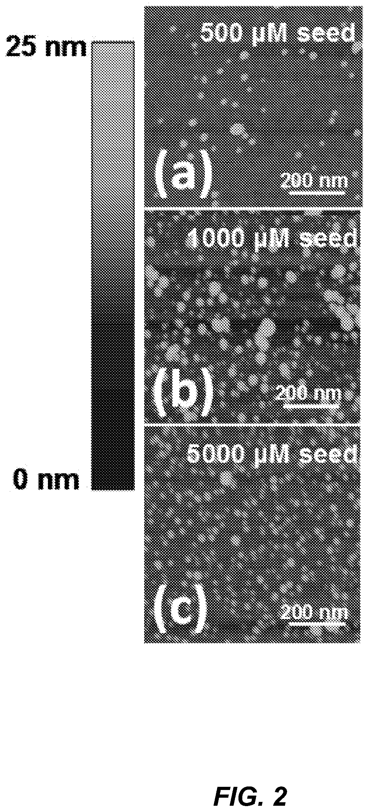

The substrate of choice was initially submersed in a solution of chloroauric acid, to which ammonium hydroxide was added. Following incubation in the seeding solution, the substrate was washed by immersion in a water bath. Immediately following the wash steps, the Au³⁺ seeded substrate was submersed into a solution of between 0.1 mM and 100 mM sodium borohydride at room temp on a linear shaker or agitated manually. The reduction was nearly immediate, and resulted in a faint pink color formed on the surface of densely seeded substrates, and was accompanied by development of a plasmon absorbance at 525 nm suggesting nanoparticle formation. AFM confirmed the presence of nanoscopic spheres with heights 5-10 nm **(****FIG. 1** view **(b)** and **FIG. 2** view **(a)).** Reduction was allowed to proceed for 1-5 minutes, followed by two submersions of the substrate in water baths. Heating of the substrate to high temperatures (> 80 °C) also caused a reduction to Au⁰ without the need for a reducing agent, likely due to decomposition of the Au-containing cluster. The Au nanoparticle-seeded substrates were moved directly from wash water baths to a solution under reducing conditions with Ag¹⁺ in solution. Growth proceeded until obvious development of the film ceased, between 15 seconds to 20 minutes. The process of silver-on-gold growth on glass slides is visually captured in **FIG. 1****,** views (a) to (e).

The initial density (seeds/mm²) and size of the seeds first deposited on the substrate was controlled by the *in situ* Au seeding step. The addition of ammonium hydroxide to chloroauric acid in basic pH may lead to ligand exchange of chloride for ammine (or amino) ligands around the Au³⁺ center, with a general form Au(NH₃)₂(H₂O)₂₋ₓ(OH)ₓ^{(3-x)+}. The resulting amine-gold complexes do not rapidly hydrolyze in basic solutions and aggregate into clusters. In moderately basic pH, the low-solubility, cationic clusters are then deposited onto negatively charged substrate surfaces. The deposited gold precipitates were subsequently reduced into Au⁰ nanoparticles by sodium borohydride in aqueous solution (step (2) above). The Au nanoparticle seeding methodology presented here is broadly applicable to a variety of substrates.

Gold cluster seeding density can be varied to obtain conditions that yielded uniform and dense seed distribution. Seeding density increased with increasing Au³⁺ concentration **(****FIG. 2** views **(a)-(c)),** which then influence the film density and morphology. Seeding of cationic Au clusters was also found to be dependent on pH, resulting in a uniform seed layer at pH 8-10.

The deposited small clusters of gold cations were reduced by NaBH₄ prior to the growth of the final cAg/Au film, without which, little silver growth was observed and the growth which occurred was uneven throughout. Following reduction of the seeds, AFM was used to confirm the presence of nanoscopic spheres with heights 5-10 nm **(****FIG. 1** view **(b)).**

Growth of the cAg/Au film was effected by reduction of silver halide onto the gold seeds. The rate of Ag¹⁺ reduction was much greater for surface-bound Ag¹⁺ ions than those in solution, and thus Ag⁰ formation was specific to the seed layer. Following washing steps after reduction, the seeded substrate was immersed in a water solution with controlled pH and temperature under reducing conditions. A solution of silver halide was added to the solution, resulting in a silver ion concentration in the range of 1 mM to 100 mM. The solution remained in growth conditions for a time between 15 seconds and 20 minutes, followed by consecutive water washes, drying, and storage under vacuum at -20°C.

The ability to chemically control gold and silver nanoparticle nano-plate sizes, as well as inter-particle spacing, allowed for optimizing the ensemble surface plasmon of the resulting film for visible and near-infrared fluorescence applications. Gold nanoparticle seed density, controlled by the HAuCl₄ concentration during precipitation/deposition, and the final nanoplate size, controlled by the seed density, growth time as well as the concentration of Au³⁺ or Ag¹⁺ ions present during the film growth step, allow relatively precise control over nano-nanoplate gap spacing. The coupling of proximal plasmonic nanoparticle nanoplates may be used as a parameter in determining both the energy of the ensemble plasmon resonance, and the magnitude of the local electric field enhancement, and thus metal-enhanced fluorescence effect, provided by the film. Similarly, the interaction at the interface between the gold seed and the silver outer coating for the cAg/Au and dAg/Au films may also be a parameter facilitating a broader plasmon and broader fluorescence enhancement than single metal films.

The synthetic conditions can be varied systematically to afford a library of plasmonic substrates. The density of Au seeds was varied by the concentration of the initial chloroauric acid. The concentration of Ag¹⁺ ions during the second step of growth determined the nano-gap distance between the raised nanostructures, ranging from isolated small nanoplates to nanoplates with 5-50 nm gaps, and to continuous rough films with narrow 1-10 nm gaps. Such solution phase synthesis method produced a library of fluorescence enhancing plasmonic (visible-NIR) cAg/Au and dAg/Au substrates with ∼5-50 nm nano-gap spacing, and plasmonic peaks in the 450-1000 nm range useful for fluorescence enhancement in the broad visible-NIR range of 400-2100 nm.

### Example 2: Seeding of gold precursors onto unmodified substrates

Preparation of Ag/Au films involved three steps: seeding of gold precursors, reduction into Au⁰ clusters, and selective growth by reduction of Ag¹⁺ **(****FIG. 1** views **(a)-(e)).** Seeding was accomplished by addition of ammonium hydroxide into a solution of chloroauric acid containing the substrate of choice. Immediately following ammonium hydroxide addition, the transparent yellow, acidic HAuCl₄ solution became cloudy and orange-yellow, with pH ∼ 9. The deposition rate of the Au³⁺ species onto the substrate was found to be rapid. Increased exposure times from one minute to twenty minutes did not significantly affect the density or size of gold seeds immobilized on the substrate.

Seeding density of Au seeds was dependent upon the initial concentration of HAuCl₄ into which the substrate was submerged prior to precipitation by ammonium hydroxide. For inorganic substrates such as glass and SiO₂, an increase of HAuCl₄ from 0.5 mM to 5 mM led to significantly increased density and uniformity of Au NP precursor seeds **(****FIG. 2** views **(a)-(c)).** Polymeric substrates such as poly(vinyl chloride), PVC, and poly(dimethylsiloxane), PDMS, required slightly higher Au³⁺ concentrations of 10 mM in order to obtain high density seeding.

### Example 3: Solution phase reduction of gold precipitate precursors

Following the deposition of Au seeds by precipitation onto the substrate of choice, the substrate was immersed into a 0.1 mM to 100 mM solution of sodium borohydride, which led to rapid formation of Au⁰ nanoparticles, evidenced by a faint pink-purple color change of the substrate. Atomic force microscopy revealed formation of Au NPs with diameters of 5-10 nm **(****FIG. 1** views **(b)),** and UV/Vis absorption spectroscopy revealed a weak surface plasmon resonance at 525 nm, typical of Au NPs in this size range.

### Example 4: Selective reduction of Ag¹⁺ onto precursor Au seeds

Submersion of the seeded substrate into an aqueous solution of silver ions under reducing conditions initiated selective reduction of Ag¹⁺ onto the seed layer, and thus the Au precursor seeds were grown into silver-on-gold plasmonic nanostructures **(****FIG. 1** view **(d)).** Silver reduction led to a color change of the substrate from pink to blue-purple, and finally a highly reflective silver color was observed on the substrate as the film thickened **(****FIG. 1** view **(c)).** SEM imaging revealed that the Ag nanostructures formed on the substrate were separated by ∼1-100 nm gap spacing, a morphology desirable for local electrical field enhancement.

### Example 5: Enhancement of fluorescent signals of TOTO-3

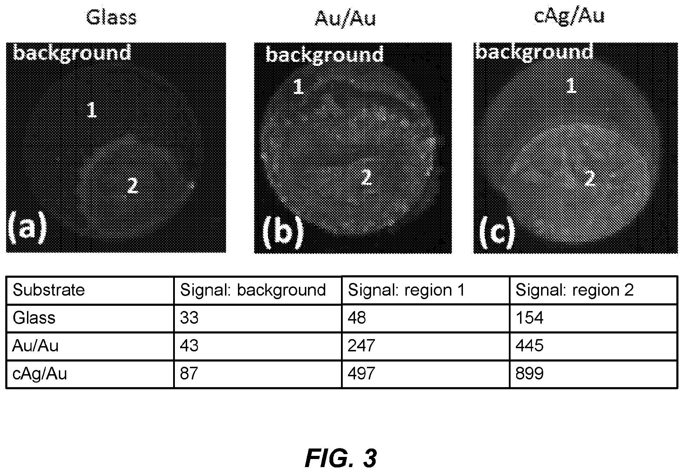

TOTO-3 is a DNA staining dye with excitation (642 nm) and emission (660 nm) similar to Cy5. A solution of TOTO-3 was pipetted onto glass, Au/Au, and cAg/Au substrates and dried in order to initially assess the fluorescence enhancement of the substrates. Compared with glass, Au/Au demonstrated greater than 10-fold increase in signal after background subtraction using the Cy5 channel of the Agilent GenePix scanner. The cAg/Au resulted in an even greater signal increase of more than 25-fold after background subtraction (**FIG. 3**). Both Au/Au and cAg/Au exhibited a much higher signal-to-noise ratio for the TOTO-3 fluorescence when compared with the glass substrate. This can be utilized for highly sensitive cell free DNA assay on the plasmonic films.

### Example 6: Quantitative analysis for cytokine assay

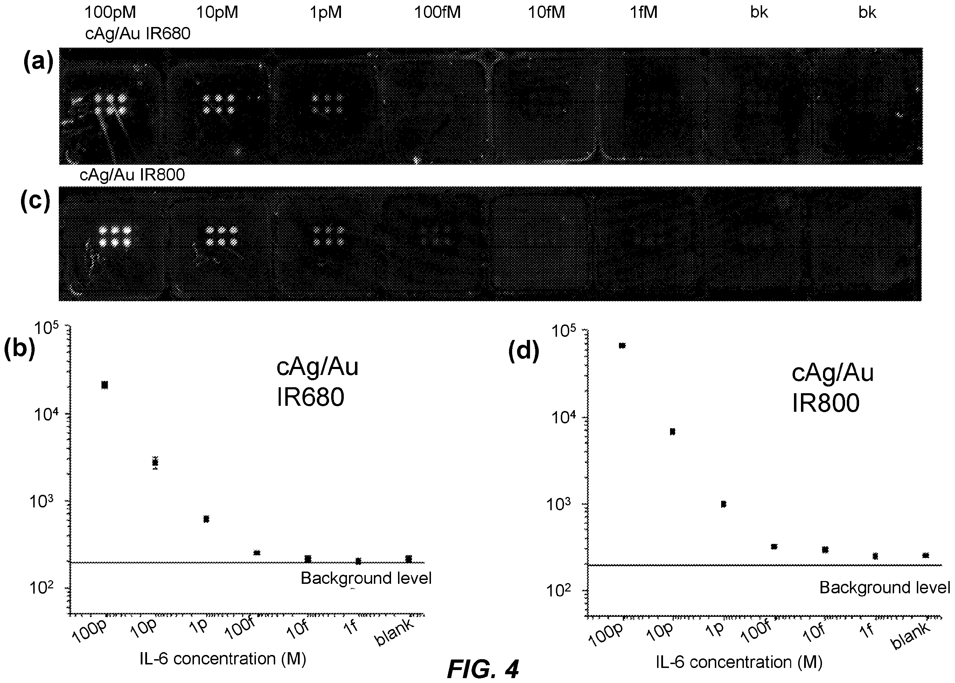

The Ag/Au film substrates for visible and NIR fluorescence enhancement applications are uniform enough to provide quantitative analysis with a dynamic range of over 6 orders of magnitude for cytokine assays (**FIG. 4**). In addition to affording biomarker quantification at low concentrations, high-throughput screening methods may benefit from the expanded dynamic range afforded by multiplexed microarray Ag/Au assays, where concentrations of analytes, as well as binding constants, may span a significant and unknown range. The present near-infrared fluorescence enhancement based upon Ag/Au films may also find additional applications as an in vitro imaging tool. For example, fluorescent agents bound to the membrane of live cells have been enhanced by Ag/Au films.

### Example 7: Quantitative analysis for Reverse Phase Protein Array assay

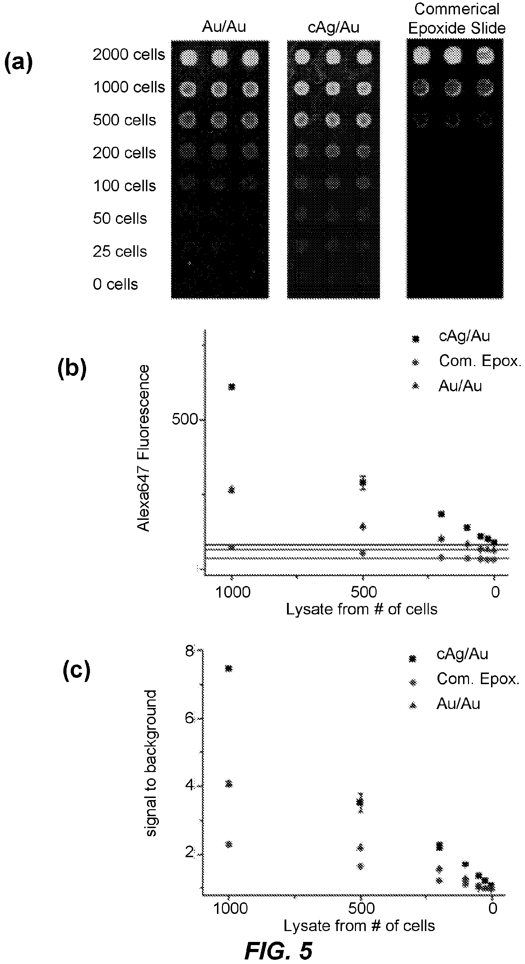

The cAg/Au film substrates for visible and NIR fluorescence enhancement applications were demonstrated for Reverse Phase Protein Array (RPPA) applications, providing quantitative analysis for the expression of human epidermal growth factor receptor 2 (HER2) in cell lines or biological tissues, using lysate from as few as 25 cells (**FIG. 5**). HER2 was labeled by anti-HER2 tagged with a visible dye, AlexaFluor647, IRDye800 and imaged with the LI-COR Odyssey Scanner. The cAg/Au film substrates demonstrated approximately 9-fold higher fluorescence signal as well as significantly higher signal to background ratio when compared with commercially available epoxy-coated slides (NEXTERION, from Schott).

### Example 8:

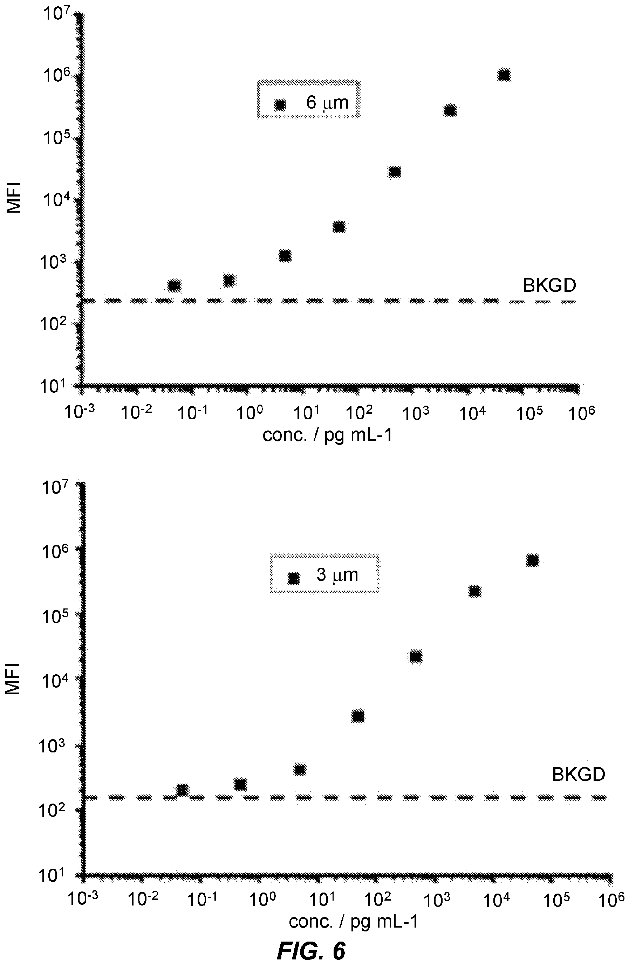

Microscopic beads were coated with a Ag/Au film of the present disclosure and used in a flow cytometry-based detection of troponin I, a marker for mycardial infarction. Results of medium fluorescence intensity (MFI) over a range of marker concentrations are provided in **Fig. 6****.** Background (BKGD) is indicated by a dashed line. Beads can be magnetic or non-magnetic.

### Example 9:

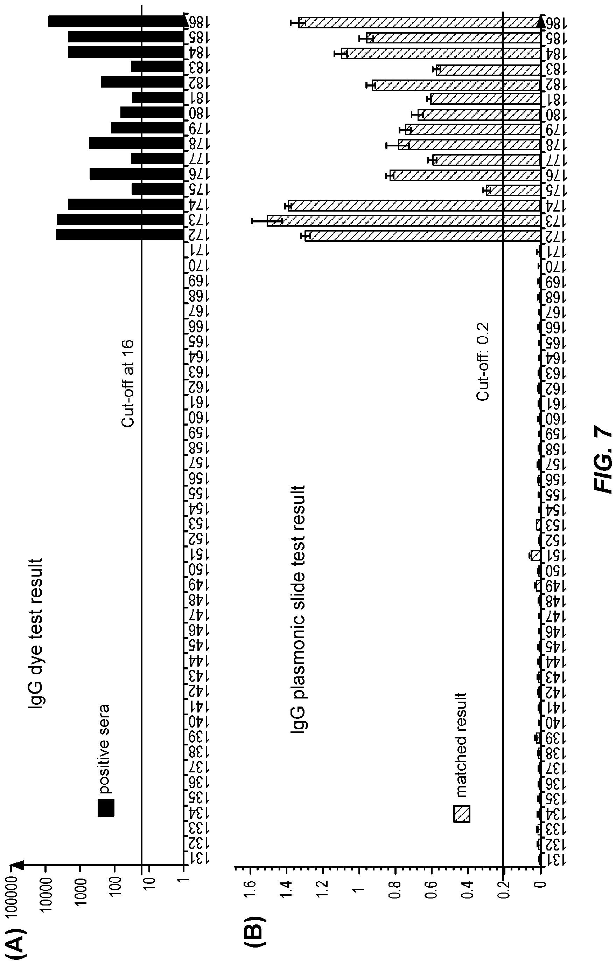

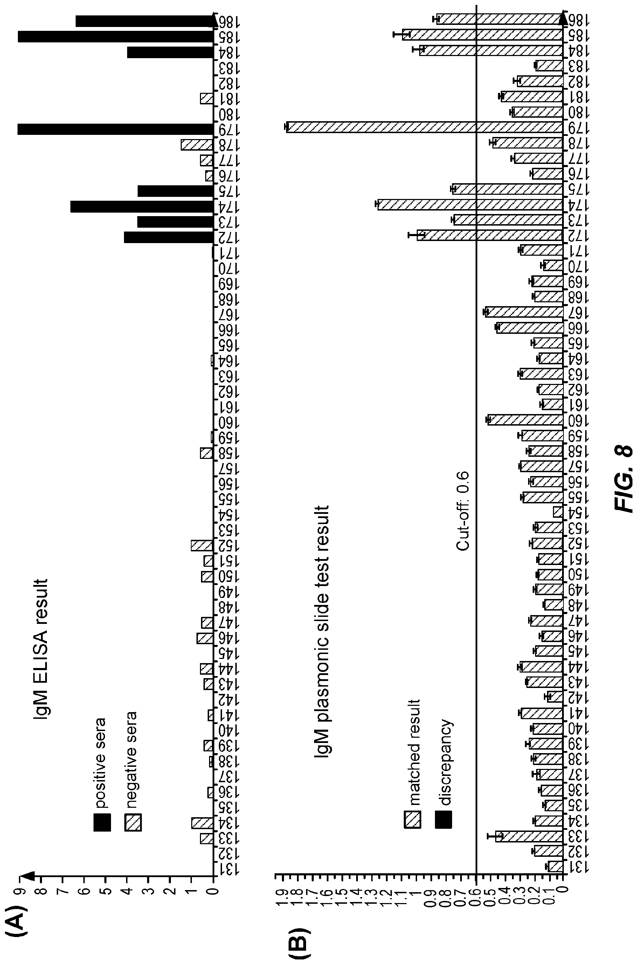

Serum samples from 56 patients (numbered 131-186) were tested for Toxoplasma antibodies using a standard IgG dye assay method without a film of the disclosure (referred to in this example as the "dye test"), and using a film of the disclosure (referred to in this example as a "plasmonic slide test"). Results for comparison are shown in FIG. 7 views (A)-(B) and FIG. 8 views (A)-(B).

A slide comprising a film of the disclosure (referred to as a "plasmonic slide") was loaded into a microarray printing robot (GESIM Nanoplotter). In this holder, spots were printed in triplicate in each well (16 wells in all per slide) for each antigen. The slides were printed to detect antigens IgG and IgM. The antigens were diluted in phosphate buffer saline (PBS) and were printed at 25 °C, resulting in microarray feature diameters of ∼300 micron. The printed slides were sealed under vacuum and stored at -80 °C until needed.

To run a sample, the printed slides were initially blocked with a buffer solution containing 1x PBS, 1mM Tris, and 5% Bovine Serum Albumin (BSA). After blocking, the slide was washed with PBS + Tween 20 solution (PBST) on an automated slide washer. The sample, human serum or whole blood, was diluted into fetal bovine serum (FBS) solution (1:100) and 100 microliter of solution was applied to a given well with printed antigens. A separate well was incubated with a positive control solution diluted 1:200 in FBS. The control solution may also be applied to each well. The solution was incubated for 1 hour at room temperature followed by washing with PBST using an automated slide washer. The array was then incubated in 4 nM IRDye800 conjugated goat anti-human IgM and 4 nM IRDye680 conjugated goat anti-human IgG in FBS for 30 min at room temperature in the dark. The plasmonic slides were then washed with an automated slide washer in PBST, followed by immersion in DI water and subsequent drying with a slide centrifuge.

Subsequently, each slide was scanned with MidaScan in the 800 mm channel. The fluorescence signal intensity was recorded, reflecting appropriate antibody in serum/whole blood/saliva.

FIG. 7 illustrates Toxo IgG titer levels based on the dye test method (A) and using the fluorescence intensity levels of plasmonic slide assay signals normalized to a control sample (B). FIG. 8 illustrates Toxo IgM titer based on the ELISA method (A) and using the fluorescence intensity levels on plasmonic slides normalized to a control sample (B). As shown in the figures, the test results based on plasmonic slide assays are highly consistent with those of the dye test.

### Example 10:

ToRCH pilot testing was performed on plasmonic slides and the test results were compared with those from commercial assays. A plasmonic slide, as in Example 9, was loaded into the microarray printing robot (GESIM Nanoplotter). In the holder, spots were printed in triplicate in each well (16 wells in all/slide) for each antigen. The slides were printed with a Rubella antigen recognized by an IgG antibody, a CMV antigen recognized by an IgG antibody, a CMV antigen recognized by an IgM antibody, and four HSV antigens. The antigens, the antibodies that recognize them, and the antibody label are listed in view (A) of FIG. 9. The antigens were diluted in phosphate buffer saline (PBS) and were printed at 25 °C, resulting in microarray feature diameters of ∼300 micron. The printed slides are sealed under vacuum and stored at -80 °C until needed.

To run a sample, the printed slides were initially blocked with a buffer solution containing 1x PBS, 1mM Tris, and 5% Bovine Serum Albumin (BSA). After blocking, the slide was washed with PBS + Tween 20 solution (PBST) on an automated slide washer. The sample, human serum or whole blood, was diluted into fetal bovine serum (FBS) solution (1:100) and 100 microliter of solution was applied to a given well with printed antigens. A separate well was incubated with a positive control solution diluted 1:200 in FBS. The control solution may also be applied to each well. The solution was incubated for 1 hour at room temperature followed by washing with PBST using an automated slide washer. The array was then incubated in 4 nM IRDye800 conjugated goat anti-human IgM and 4 nM IRDye680 conjugated goat anti-human IgG in FBS for 30 min at room temperature in the dark. The plasmonic slides were then washed with an automated slide washer in PBST, followed by immersion in DI water and subsequent drying with a slide centrifuge.

Subsequently, each slide was scanned with MidaScan in the 800 mm channel. The fluorescence signal intensity was recorded, reflecting appropriate antibody in serum/whole blood/saliva.

FIG. 9 view (A) shows a correlation between ELISA tests for ToRCH panel antibodies and preliminary plasmonic slide ToRCH assay. In this initial testing, several different printed antigens were used to detect the same HSV antibody in order to determine the relative effectiveness of antigens manufactured by different venders. The column % represents the correlation of plasmonic slide assay with the following tests: antibodies against Rubella tested with Architect ABBOTT (Abbott Laboratories, Wiesbaden, Germany); IgG and IgM against CMV and HSV tested with Evolis (Bio-Rad Laboratories, Hercules, CA). Commercial tests were conducted in accordance with manufacturer instructions. The cut-off value represents the normalized intensity of the signal compared with a positive control sample. FIG. 9 view (B) schematically illustrates the layout of the printed microarray slide (spot spacing is at 1 mm), along with a scanned image of a patient (shown in FIG.9 view (C)). The key to the right side of the figures indicates the printed antigens corresponding to those in the schematic.

### Example 11:

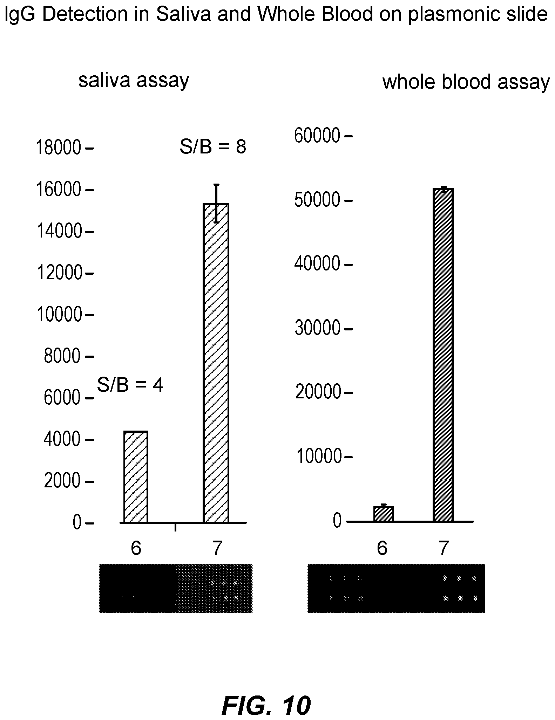

Plasmonic slide assay was used to detect toxoplasmosis IgG in saliva and whole blood samples. For a saliva assay, the protocol is substantially the same as that for whole blood samples. Saliva is retrieved from a person by spitting into an Eppendorf tube, and the sample is centrifuged to remove any particles. The same protocol as described above in Examples 9 and 10 was then followed for conducting the assays. FIG. 10 illustrates assay results of anti-toxoplasmosis IgG in the saliva and whole blood samples of two people, with detection on plasmonic slides. These results show successful detection of toxoplasmosis IgG in saliva samples, matching the data obtained with serum and blood.

### Example 12:

Beads in the 0.01 to 10 micron size range are coated with a film in accordance with the present disclosure, comprising nanostructures comprising silver-on-gold nanoparticles (referred to in this example as "plasmonic beads"). The beads may or may not have a magnetic iron oxide core. The beads have a surface that is functionalized with binding elements for the capture of exosomes or cell-free DNA. The beads may be imparted with a magnetic property to facilitate capture and washing of exosomes or cell-free DNA. The captured exosomes or cell-free DNA is analyzed while on the plasmonic beads using a fluorescence microscope, a fluorimeter, or flow cytometry. For exosomes, detection comprises attaching a fluorescently labeled detection antibody specific for an antigen on the exosomes. For cell-free DNA, detection comprises labeling the cell-free DNA with an intercalating dye.

While preferred embodiments of the present invention have been shown and described herein, it will be obvious to those skilled in the art that such embodiments are provided by way of example only, and the scope of the invention is defined by the claims. Numerous variations, changes, and substitutions will now occur to those skilled in the art without departing from the invention as defined in the claims. It should be understood that various alternatives to the embodiments of the invention described herein may be employed in practicing the invention, within the scope of the claims. It is intended that the following claims define the scope of the invention and that methods and structures within the scope of these claims and their equivalents be covered thereby.

## Claims

1. A film comprising raised nanostructures on a substrate, wherein:
the nanostructures of the film comprise silver-on-gold nanoparticles; and
the nanostructures are separated from one another by gaps, wherein the gaps have widths between 5 nm to 50 nm, and lengths between 5 nm and 1000 nm.

2. The film of claim 1, wherein:
the nanostructures have an average width and length between 50 nm to 500 nm, such as an average width and length between 100 nm to 200 nm;
the film has a nanoplate size of between 1000 nm² to 250,000 nm²;
the film comprises irregular features and a heterogenous structure; or
the height of the film is between 5 nm and 500 nm.

3. The film of claim 1 or claim 2, wherein the film is quasi-continuous through a percolating path and conducting based on electron microscopy imaging and electrical conductivity; or wherein the film is discontinuous based on electron microscopy imaging and electrical conductivity.

4. The film of any preceding claim, wherein the substrate comprises one or more materials selected from the group consisting of: glass, polystyrene, quartz, silica, nylon, nitrocellulose, polyvinyl chloride, polydopamine, polydimethyl siloxane, polyvinylidene fluoride, silicon, silicon dioxide, a polymer, iron oxide, and a plastic.

5. The film of any preceding claim, wherein the substrate comprises a flat surface, a curved surface, a spherical surface, a well in a multi-well plate, or a three-dimensional porous membrane.

6. The film of any preceding claim, wherein the substrate is a bead with or without a magnetic core; optionally wherein the bead has a diameter ranging from 0.05 microns to 200 microns; optionally wherein the bead is in a container, such as a well in a 96-well plate or a 384-well plate.

7. The film of any preceding claim, further comprising an array of binding elements on the film, wherein the binding elements bind to an analyte; optionally wherein the array of binding elements comprises a plurality of different binding elements, each of which binds a different analyte.

8. The film of claim 7, wherein the binding elements are selected from the group consisting of proteins, antibodies, antigen-binding antibody fragments, cells, aptamers, peptides, polynucleotides, exosomes, and tissue slices;
such as wherein the binding elements are antigens for detecting antibodies in a sample; optionally wherein the antigens bind one or more antibodies selected from the group consisting of: total human IgG, IgM, IgA and IgE; anti-HLA antibodies; anti-dsDNA antibodies; anti-Smith antibodies; antibodies diagnostic of Systemic Lupus Erythematosus (SLE), such as anti-nucleosome, anti U1RNP, and anti-PO antibodies; antibodies diagnostic of cardiovascular disease; antibodies diagnostic of Toxoplasmosis, Rubella, Rabies, Dengue, Malaria, lyme disease, African Trypanosomiasis, cholera, cryptosporidiosis, dengue, influenza, Japanese Encephalitis, Leishmaniasis, measles, meningitis, onchocerciasis, pneumonia, tuberculosis, typhoid, or yellow fever; antibodies specific for CMV, HSV-1/2, HBA, HBV, HCV, HDV, HIV; HPV, Ebola virus, rotavirus, human leukocyte antigens, Thyroid Stimulating Hormone Receptor (TSHR), thyroperoxidate, Thyroglobulin, tissue transglutaminase (tTG), endomysium, deamidated gliadin peptide; and antibodies specific for tumor-associated antigens selected from p53, NY-ESO-1, MAGE A4, HuD, CAGE, GBU4-5, and SOX2; or wherein the antigens comprise (a) one or more Toxoplasmosis gondii antigens, one or more Rubella antigens, one or more CMV antigens, and one or more HSV antigens; and optionally (b) one or more syphilis antigens and one or more HIV antigens.

9. The film of claim 7, wherein the analyte is a protein, an antibody, a peptide, a nucleic acid, an enzyme, a cell, an exosome, a cell free DNA, or a tissue;
optionally wherein the analyte is a biomarker for a condition of a subject; such as wherein the analyte is an inflammatory cytokine, a biomarker for cardiovascular disease, a biomarker for infectious disease, a biomarker for an inflammatory bowel disease, or a biomarker for cancer;
such as wherein (a) the analyte is a biomarker for cardiovascular disease selected from troponin I, c-reactive protein (CRP), NT-ProBNP, and an antibody to Annexin A5, SDHA, ATP1A3, titin, myosin, ADBRK, EDNRA, EDNRB, AGTR1, CHRM2, or HSPD; (b) the analyte is a biomarker of infectious disease selected from hepatitis B virus (HBV) core antigen, HBV surface antigen, Dengue NS1 antigen, and antibodies to one or more of Toxoplasmosis gondii, Rubella, CMV, HCV, HIV, syphilis, and HSV; or (c) the analyte is a biomarker for cancer selected from prostate-specific antigen (PSA), carcinoembryonic antigen (CEA), cancer antigen-125 (CA125), AFP, SCC, CA19-9, CA242, NSE, Cyfa21-1, CA15-3, and total T-PSA.

10. A method of detecting an analyte, the method comprising:
providing a film comprising raised nanostructures on a substrate, wherein nanostructures of the film comprise silver-on-gold nanoparticles, and the nanostructures are separated from one another by gaps; wherein the gaps have widths between 5 nm to 50 nm, and lengths between 5 nm and 1000 nm; applying to the film an analyte and a label for the analyte, wherein the label comprises a fluorophore; and
detecting the analyte by detecting a fluorescent signal of the fluorophore, wherein intensity of the fluorescent signal is enhanced relative to the fluorescent signal of the fluorophore in the absence of the film;
and optionally further comprising determining concentration, identity, or location of the analyte based on detecting the fluorescent signal.

11. The method of claim 10, wherein
the fluorescent signal is in the range of 400 nm to 2100 nm; or
the fluorescent signal is enhanced for fluorophores within 1000 nm of the surface of the film; or
the fluorophore is a near-infra-red fluorophore having an emission of about 700 nm to about 800 nm, and the intensity of the fluorescent signal is enhanced by at least 30-fold; or
the fluorophore is a visible dye having an emission of about 400 nm to about 700 nm, and the intensity of the fluorescent signal is increased by at least 3-fold.

12. The method of claim 10 or claim 11, wherein the analyte is on a surface, such as wherein the analyte is bound to the surface by a binding element conjugated to the surface; optionally wherein the surface is a DNA microarray, an RNA microarray, a miRNA microarray, a peptide microarray, an antigen microarray, a protein microarray, or an antibody microarray.

13. The method of any of claims 10-12, wherein the analyte is an inflammatory cytokine, a biomarker for cardiovascular disease, a biomarker for infectious disease, or a biomarker for cancer; such as wherein (a) the analyte is a biomarker for cardiovascular disease selected from troponin I, c-reactive protein (CRP), NT-ProBNP, and an antibody to Annexin A5, SDHA, ATP1A3, titin, myosin, ADBRK, EDNRA, EDNRB, AGTR1, CHRM2, or HSPD; (b) the analyte is a biomarker of infectious disease selected from hepatitis B virus (HBV) core antigen, HBV surface antigen, Dengue NS1 antigen, and antibodies to one or more of Toxoplasmosis gondii, Rubella, HCV, HSV, HIV, syphilis, and CMV; or (c) the analyte is a biomarker for cancer selected from prostate-specific antigen (PSA), carcinoembryonic antigen (CEA), cancer antigen-125 (CA125), AFP, SCC, CA19-9, CA242, NSE, Cyfa21-1, CA15-3, and total T-PSA.

14. The method of any of claims 10-13, wherein the film further comprises an array of binding elements on the film, wherein the binding elements bind to the analyte.

15. The method of Claim 14, wherein the binding elements are a plurality of antigens for detecting antibodies in a sample, wherein the antigens are on a surface of the film; and wherein the method comprises:
contacting the plurality of antigens with a sample and one or more labels for detecting the one or more antibodies bound to an antigen of the plurality of antigens, wherein each label comprises a fluorophore; and
detecting the one or more bound antibodies by detecting a fluorescent signal of the fluorophore, wherein intensity of the fluorescent signal is enhanced relative to the fluorescent signal of the fluorophore in the absence of the film.

16. The method of claim 15, wherein the one or more antibodies are IgG antibodies, and the one or more labels are anti-IgG antibodies;
or wherein (i) the one or more antibodies comprise one or more antibody subtypes selected from IgG, IgM, IgA, and IgE; (ii) the one or more labels comprise a plurality of corresponding label antibodies selected from anti-IgG, anti-IgM, anti IgA, and anti-IgE antibodies; (iii) the fluorophore of a label antibody corresponding to one subtype is different from the fluorophore corresponding to any other subtype, such as wherein the fluorophores are selected from Cy3, Cy5, IR680, and IR800; and (iv) the method further comprises identifying or quantifying the different subtypes based on the fluorescent signal.

## Patentansprüche

1. Film, der erhabene Nanostrukturen auf einem Substrat umfasst, wobei:
die Nanostrukturen des Films Silber-auf-Gold-Nanopartikel umfassen; und
die Nanostrukturen durch Lücken voneinander getrennt sind, wobei die Lücken eine Breite zwischen 5 nm und 50 nm und eine Länge zwischen 5 nm und 1000 nm haben.

2. Film nach Anspruch 1, wobei:
die Nanostrukturen eine durchschnittliche Breite und Länge zwischen 50 nm und 500 nm, wie z.B. eine durchschnittliche Breite und Länge zwischen 100 nm und 200 nm haben;
der Film eine Nanoplattengröße zwischen 1000 nm² und 250.000 nm² hat;
der Film unregelmäßige Merkmale und eine heterogene Struktur hat; oder die Höhe des Films zwischen 5 nm und 500 nm liegt.

3. Film nach Anspruch 1 oder Anspruch 2, wobei der Film durch einen Perkolationspfad quasikontinuierlich und leitfähig auf der Basis von Elektronenmikroskopieabbildung und elektrischer Leitfähigkeit ist; oder wobei der Film diskontinuierlich auf der Basis von Elektronenmikroskopieabbildung und elektrischer Leitfähigkeit ist.

4. Film nach einem vorherigen Anspruch, wobei das Substrat ein oder mehrere Materialien umfasst, ausgewählt aus der Gruppe bestehend aus: Glas, Polystyrol, Quarz, Silika, Nylon, Nitrocellulose, Polyvinylchlorid, Polydopamin, Polydimethylsiloxan, Polyvinylidenfluorid, Silizium, Siliziumdioxid, einem Polymer, Eisenoxid und einem Kunststoff.

5. Film nach einem vorherigen Anspruch, wobei das Substrat eine flache Oberfläche, eine gekrümmte Oberfläche, eine sphärische Oberfläche, ein Well in einer Multiwellplatte oder eine dreidimensionale poröse Membran umfasst.

6. Film nach einem vorherigen Anspruch, wobei das Substrat ein Kügelchen mit oder ohne Magnetkern ist; wobei das Kügelchen optional einen Durchmesser im Bereich von 0,05 Mikron bis 200 Mikron hat; wobei sich das Kügelchen optional in einem Behälter, wie z.B. einem Well in einer 96-Well-Platte oder einer 384-Well-Platte, befindet.

7. Film nach einem vorherigen Anspruch, ferner umfassend ein Array von Bindungselementen auf dem Film, wobei sich die Bindungselemente an einen Analyten binden; wobei das Array von Bindungselementen optional eine Mehrzahl unterschiedlicher Bindungselemente umfasst, die jeweils einen unterschiedlichen Analyten binden.

8. Film nach Anspruch 7, wobei die Bindungselemente ausgewählt sind aus der Gruppe bestehend aus Proteinen, Antikörpern, antigenbindenden Antikörperfragmenten, Zellen, Aptameren, Peptiden, Polynucleotiden, Exosomen und Gewebeschnitten;
z.B. wobei die Bindungselemente Antigene zum Detektieren von Antikörpern in einer Probe sind; wobei die Antigene optional einen oder mehrere Antikörper binden, die ausgewählt sind aus der Gruppe bestehend aus: humanem Gesamt-IgG, -IgM, -IgA und -IgE; Anti-HLA-Antikörpern; Anti-dsDNA-Antikörpern; Anti-Smith-Antikörpern; Antikörpern zur Diagnose des systemischen Lupus erythematodes (SLE), wie Anti-Nucleosom-, Anti-U1RNP- und Anti-PO-Antikörper; Antikörpern zur Diagnose einer kardiovaskulären Erkrankung; Antikörpern zur Diagnose von Toxoplasmose, Röteln, Tollwut, Dengue, Malaria, Lyme-Krankheit, Afrikanischer Trypanomiasis, Cholera, Kryptosporidiose, Dengue, Influenza, Japanischer Enzephalitis, Leishmaniase, Masern, Meningitis, Onchozerkose, Pneumonie, Tuberkulose, Typhus oder Gelbfieber; Antikörpern, die spezifisch sind für CMV, HSV-1/2, HBA, HBV, HCV, HDV, HIV, HPV, Ebola-Virus, Rotavirus, humane Leukozyten-Antigene, thyreoidstimulierenden Hormonrezeptor (TSHR), Thyreoperoxidat, Thyreoglobulin, Gewebetransglutaminase (tTG), Endomysium, deamidiertes Gliadinpeptid; und Antikörpern, die für tumorassoziierte Antigene spezifisch sind, ausgewählt aus p53, NY-ESO-1, MAGE A4, HuD, CAGE, GBU4-5 und SOX2; oder wobei die Antigene (a) ein oder mehrere Toxoplasmosis gondii-Antigene, ein oder mehrere Röteln-Antigene, ein oder mehrere CMV-Antigene und ein oder mehrere HSV-Antigene; und optional (b) ein oder mehrere Syphilis-Antigene und ein oder mehrere HIV-Antigene umfassen.

9. Film nach Anspruch 7, wobei der Analyt ein Protein, ein Antikörper, ein Peptid, eine Nucleinsäure, ein Enzym, eine Zelle, ein Exosom, eine zellfreie DNA oder ein Gewebe ist;
wobei der Analyt optional ein Biomarker für einen Zustand eines Subjekts ist; z.B. wobei der Analyt ein inflammatorisches Zytokin, ein Biomarker für eine kardiovaskuläre Erkrankung, ein Biomarker für eine Infektionskrankheit, ein Biomarker für eine entzündliche Darmerkrankung oder ein Biomarker für Krebs ist;
z.B. wobei (a) der Analyt ein Biomarker für eine kardiovaskuläre Erkrankung ist, ausgewählt aus Troponin I, C-reaktivem Protein (CRP), NT-ProBNP und einem Antikörper gegen Annexin A5, SDHA, ATP1A3, Titin, Myosin, ADBRK, EDNRA, EDNRB, AGTR1, CHRM2 oder HSPD; (b) der Analyt ein Biomarker für eine Infektionskrankheit ist, ausgewählt aus Hepatitis-B-Virus-(HBV)-Kernantigen, HBV-Oberflächenantigen, Dengue-NSl-Antigen und Antikörpern gegen eines oder mehrere aus Toxoplasmosis gondii, Röteln, CMV, HCV, HIV, Syphilis und HSV; oder (c) der Analyt ein Biomarker für Krebs ist, ausgewählt aus Prostataspezifischem Antigen (PSA), karzinoembryonalem Antigen (CEA), Krebsantigen-125 (CA125), AFP, SCC, CA19-9, CA242, NSE, Cyfa21-1, CA15-3 und Gesamt- T-PSA.

10. Verfahren zum Detektieren eines Analyten, wobei das Verfahren Folgendes beinhaltet:
Bereitstellen eines Films, der erhabene Nanostrukturen auf einem Substrat umfasst, wobei die Nanostrukturen des Films Silber-auf-Gold-Nanopartikel umfassen und die Nanostrukturen durch Lücken voneinander getrennt sind; wobei die Lücken eine Breite zwischen 5 nm und 50 nm und eine Länge zwischen 5 nm und 1000 nm haben; Aufbringen eines Analyten und einer Markierung für den Analyten auf den Film, wobei die Markierung ein Fluorophor umfasst; und
Detektieren des Analyten durch Detektieren eines Fluoreszenzsignals des Fluorophors, wobei die Intensität des Fluoreszenzsignals relativ zum Fluoreszenzsignal des Fluorophors in Abwesenheit des Films verstärkt ist;
und optional ferner Bestimmen der Konzentration, Identität oder Lage des Analyten auf der Basis des Detektierens des Fluoreszenzsignals.

11. Verfahren nach Anspruch 10, wobei
das Fluoreszenzsignal im Bereich von 400 nm bis 2100 nm liegt; oder
das Fluoreszenzsignal für Fluorophore innerhalb von 1000 nm von der Oberfläche des Films verstärkt ist;
oder das Fluorophor ein Nahinfrarot-Fluorophor mit einer Emission von etwa 700 nm bis etwa 800 nm ist und die Intensität des Fluoreszenzsignals um wenigstens das 30-fache verstärkt ist; oder
das Fluorophor ein sichtbarer Farbstoff mit einer Emission von etwa 400 nm bis etwa 700 nm ist und die Intensität des Fluoreszenzsignals um wenigstens das 3-fache erhöht ist.

12. Verfahren nach Anspruch 10 oder Anspruch 11, wobei sich der Analyt auf einer Oberfläche befindet, z.B. wobei der Analyt durch ein an die Oberfläche konjugiertes Bindungselement an die Oberfläche gebunden ist;
wobei die Oberfläche optional ein DNA-Microarray, ein RNA-Microarray, ein miRNA-Microarray, ein Peptid-Microarray, ein Antigen-Microarray, ein Protein-Microarray oder ein Antikörper-Microarray ist.

13. Verfahren nach einem der Ansprüche 10-12, wobei der Analyt ein inflammatorisches Zytokin, ein Biomarker für eine kardiovaskuläre Erkrankung, ein Biomarker für eine Infektionskrankheit oder ein Biomarker für Krebs ist; z.B. wobei (a) der Analyt ein Biomarker für eine kardiovaskuläre Erkrankung ist, ausgewählt aus Troponin I, C-reaktivem Protein (CRP), NT-ProBNP und einem Antikörper gegen Annexin A5, SDHA, ATP1A3, Titin, Myosin, ADBRK, EDNRA, EDNRB, AGTR1, CHRM2 oder HSPD; (b) der Analyt ein Biomarker für eine Infektionskrankheit ist, ausgewählt aus Hepatitis-B-Virus-(HBV)-Kernantigen, HBV-Oberflächenantigen, Dengue-NSl-Antigen und Antikörpern gegen eines oder mehrere aus Toxoplasmosis gondii, Röteln, HCV, HSV, HIV, Syphilis und CMV; oder (c) der Analyt ein Biomarker für Krebs ist, ausgewählt aus Prostataspezifischem Antigen (PSA), karzinoembryonalem Antigen (CEA), Krebsantigen-125 (CA125), AFP, SCC, CA19-9, CA242, NSE, Cyfa21-1, CA15-3 und Gesamt- T-PSA.

14. Verfahren nach einem der Ansprüche 10-13, wobei der Film ferner ein Array von Bindungselementen auf dem Film umfasst, wobei sich die Bindungselemente an den Analyten binden.

15. Verfahren nach Anspruch 14, wobei die Bindungselemente eine Mehrzahl von Antigenen zum Detektieren von Antikörpern in einer Probe sind, wobei sich die Antigene auf einer Oberfläche des Films befinden; und
wobei das Verfahren Folgendes beinhaltet:
Inkontaktbringen der Mehrzahl von Antigenen mit einer Probe und einer oder mehreren Markierungen zum Detektieren der ein oder mehreren Antikörper, die an ein Antigen der Mehrzahl von Antigenen gebunden sind, wobei jede Markierung ein Fluorophor umfasst; und
Detektieren der ein oder mehreren gebundenen Antikörper durch Detektieren eines Fluoreszenzsignals des Fluorophors, wobei die Intensität des Fluoreszenzsignals relativ zum Fluoreszenzsignal des Fluorophors in Abwesenheit des Films verstärkt ist.

16. Verfahren nach Anspruch 15, wobei die ein oder mehreren Antikörper IgG-Antikörper sind und die ein oder mehreren Markierungen Anti-IgG-Antikörper sind;
oder wobei (i) die ein oder mehreren Antikörper einen oder mehrere Antikörper-Subtypen umfassen, die aus IgG, IgM, IgA und IgE ausgewählt sind; (ii) die ein oder mehreren Markierungen eine Mehrzahl von entsprechenden Markierungsantikörpern umfassen, die aus Anti-IgG-, Anti-IgM-, Anti-IgA- und Anti-IgE-Antikörpern ausgewählt sind; (iii) das Fluorophor eines Markierungsantikörpers, das einem Subtyp entspricht, sich von dem Fluorophor unterscheidet, das irgendeinem anderen Subtyp entspricht, z.B. wobei die Fluorophore aus Cy3, Cy5, IR680 und IR800 ausgewählt sind; und (iv) das Verfahren ferner das Identifizieren oder Quantifizieren der verschiedenen Subtypen anhand des Fluoreszenzsignals beinhaltet.

## Revendications

1. Film comprenant des nanostructures surélevées sur un substrat, dans lequel :
les nanostructures du film comprennent des nanoparticules d'or revêtues d'argent ; et
les nanostructures sont séparées les unes des autres par des espaces, les espaces ayant des largeurs de 5 nm à 50 nm, et des longueurs de 5 nm à 1 000 nm.

2. Film selon la revendication 1, dans lequel :
les nanostructures ont une largeur et une longueur moyennes de 50 nm à 500 nm, par exemple une largeur et une longueur moyennes de 100 nm à 200 nm ;
le film ayant une taille de nanofeuille de 1 000 nm² à 250 000 nm² ;
le film comprenant des caractéristiques irrégulières et une structure hétérogène ; ou
la hauteur du film étant de 5 nm à 500 nm.

3. Film selon la revendication 1 ou la revendication 2, le film étant quasi-continu à travers un chemin de percolation et conducteur sur la base d'une imagerie par microscopie électronique et de la conductivité électrique ; ou le film étant discontinu sur la base d'une imagerie par microscopie électronique et de la conductivité électrique.

4. Film selon l'une quelconque des revendications précédentes, dans lequel le substrat comprend un ou plusieurs matériaux sélectionnés dans le groupe constitué : du verre, du polystyrène, du quartz, de la silice, du nylon, de la nitrocellulose, du chlorure de polyvinyle, de la polydopamine, du polydiméthylsiloxane, du fluorure de polyvinylidène, du silicium, du dioxyde de silicium, d'un polymère, de l'oxyde de fer et d'un plastique.

5. Film selon l'une quelconque des revendications précédentes, dans lequel le substrat comprend une surface plate, une surface courbe, une surface sphérique, un puits dans une plaque multi-puits, ou une membrane poreuse tridimensionnelle.

6. Film selon l'une quelconque des revendications précédentes, dans lequel le substrat est une perle avec ou sans noyau magnétique ; optionnellement dans lequel la perle a un diamètre de 0,05 micron à 200 microns ; optionnellement dans lequel la perle se trouve dans un contenant, par exemple un puits dans une plaque de 96 puits ou une plaque de 384 puits.

7. Film selon l'une quelconque des revendications précédentes, comprenant en outre un réseau d'éléments de liaison sur le film, les éléments de liaison se liant à un analyte ; optionnellement dans lequel le réseau d'éléments de liaison comprend une pluralité de différents éléments de liaison, chacun d'entre eux se liant à un différent analyte.

8. Film selon la revendication 7, dans lequel les éléments de liaison sont sélectionnés dans le groupe constitué de protéines, d'anticorps, de fragments d'anticorps se liant à des antigènes, de cellules, d'aptamères, de peptides, de polynucléotides, d'exosomes et de tranches de tissus ;
par exemple dans lequel les éléments de liaison sont des antigènes pour la détection d'anticorps dans un échantillon ; optionnellement dans lequel les antigènes se lient à un ou plusieurs anticorps sélectionnés dans le groupe constitué de : IgG humaine totale, IgM, IgA et IgE ; anticorps anti-HLA ; anticorps anti-ADNdb ; anticorps anti-Smith ; anticorps diagnostiques pour le lupus érythémateux disséminé (LED), tels que les anticorps anti-nucléosome, anti U1RNP, et anti-P0 ; anticorps diagnostiques pour des maladies cardiovasculaires ; anticorps diagnostiques pour la toxoplasmose, la rubéole, la rage, la dengue, le paludisme, la maladie de Lyme, la trypanosomatose africaine, le choléra, la cryptosporidiose, la dengue, la grippe, l'encéphalite japonaise, la leishmaniose, la rougeole, la méningite, l'onchocercose, la pneumonie, la tuberculose, la typhoïde ou la fièvre jaune ; anticorps spécifiques pour le CMV, le HSV-1/2, l'antigène HB, le VHB, le VHC, le VHD, le VIH ; VPH, virus Ebola, rotavirus, antigènes leucocytaires humains, récepteur de la thyréostimuline (TSHR), thyroperoxydate, thyroglobuline, transglutaminase tissulaire (tTG), endomysium, peptide de gliadine désamidée ; et anticorps spécifiques pour des antigènes associés à des tumeurs sélectionnés parmi p53, NY-ESO-1, MAGE A4, HuD, CAGE, GBU4-5 et SOX2 ; ou dans lequel les antigènes comprennent (a) un ou plusieurs antigènes de la toxoplasmose gondii, un ou plusieurs antigènes de la rubéole, un ou plusieurs antigènes du CMV, et un ou plusieurs antigènes du HSV ; et, optionnellement (b), un ou plusieurs antigènes de la syphilis et un ou plusieurs antigènes du VIH.

9. Film selon la revendication 7, dans lequel l'analyte est une protéine, un anticorps, un peptide, un acide nucléique, une enzyme, une cellule, un exosome, un ADN acellulaire, ou un tissu ;
optionnellement dans lequel l'analyte est un biomarqueur pour une affection d'un sujet ; par exemple l'analyte est une cytokine inflammatoire, un biomarqueur pour une maladie cardiovasculaire, un biomarqueur pour une maladie infectieuse, un biomarqueur pour une maladie inflammatoire intestinale, ou un biomarqueur pour un cancer ;
par exemple dans lequel (a) l'analyte est un biomarqueur pour une maladie cardiovasculaire sélectionné parmi la troponine I, la protéine C-réactive (CRP), NT-ProBNP et un anticorps dirigé contre l'Annexine A5, SDHA, ATP1A3, la titine, la myosine, ADBRK, EDNRA, EDNRB, AGTR1, CHRM2, ou HSPD ; (b) l'analyte est un biomarqueur pour une maladie infectieuse sélectionné parmi l'antigène de la nucléocapside du virus de l'hépatite B (VHB), l'antigène de surface du VHB, l'antigène NS1 de la dengue, et des anticorps dirigés contre un ou plusieurs de la toxoplasmose gondii, de la rubéole, du CMV, du VHC, du VIH, de la syphilis et du HSV ; ou (c) l'analyte est un biomarqueur pour un cancer sélectionné parmi l'antigène spécifique de la prostate (PSA), l'antigène carcinoembryonnaire (CEA), l'antigène tumoral 125 (CA125), l'AFP, SCC, CA19-9, CA242, NSE, Cyfa21-1, CA15-3, et PSA total T-PSA.

10. Procédé de détection d'un analyte, le procédé comprenant :
la fourniture d'un film comprenant des nanostructures surélevées sur un substrat, dans lequel les nanostructures du film comprennent des nanoparticules d'or revêtues d'argent, et les nanostructures sont séparées les unes des autres par des espaces ; dans lequel les espaces ont des largeurs de 5 nm à 50 nm, et des longueurs de 5 nm à 1 000 nm ;
l'application sur le film d'un analyte et d'un marqueur pour l'analyte, le marqueur comprenant un fluorophore ; et
la détection de l'analyte par détection d'un signal fluorescent du fluorophore, l'intensité du signal fluorescent étant augmentée par rapport au signal fluorescent du fluorophore en l'absence de film ;
et comprenant optionnellement en outre une détermination de la concentration, de l'identité ou de l'emplacement de l'analyte en fonction de la détection du signal fluorescent.

11. Procédé selon la revendication 10, dans lequel
le signal fluorescent est dans une longueur d'onde de 400 nm à 2 100 nm ; ou
le signal fluorescent est augmenté pour les fluorophores qui se trouvent à 1 000 nm au maximum de la surface du film ; ou
le fluorophore est un fluorophore qui émet dans l'infrarouge proche à une longueur d'onde d'environ 700 nm à environ 800 nm, et l'intensité du signal fluorescent est multipliée au moins par 30 ; ou
le fluorophore est un colorant visible qui émet à une longueur d'onde d'environ 400 nm à environ 700 nm, et l'intensité du signal fluorescent est multipliée au moins par 3.

12. Procédé selon la revendication 10 ou la revendication 11, dans lequel l'analyte se trouve sur une surface, par exemple dans lequel l'analyte est lié à la surface par un élément de liaison conjugué à la surface ;
optionnellement dans lequel la surface est un microréseau d'ADN, un microréseau d'ARN, un microréseau de miARN, un microréseau de peptides, un microréseau d'antigènes, un microréseau de protéines, ou un microréseau d'anticorps.

13. Procédé selon l'une quelconque des revendications 10 à 12, dans lequel l'analyte est une cytokine inflammatoire, un biomarqueur pour une maladie cardiovasculaire, un biomarqueur pour une maladie infectieuse, ou un biomarqueur pour un cancer ; par exemple dans lequel (a) l'analyte est un biomarqueur pour une maladie cardiovasculaire sélectionné parmi la troponine I, la protéine C-réactive (CRP), NT-ProBNP et un anticorps dirigé contre l'Annexine A5, SDHA, ATP1A3, la titine, la myosine, ADBRK, EDNRA, EDNRB, AGTR1, CHRM2, ou HSPD ; (b) l'analyte est un biomarqueur pour une maladie infectieuse sélectionné parmi l'antigène de la nucléocapside du virus de l'hépatite B (VHB), l'antigène de surface du VHB, l'antigène NS1 de la dengue, et des anticorps dirigés contre un ou plusieurs de la toxoplasmose gondii, de la rubéole, du VHC, du HSV, du VIH, de la syphilis et du CMV ; ou (c) l'analyte est un biomarqueur pour un cancer sélectionné parmi l'antigène spécifique de la prostate (PSA), l'antigène carcinoembryonnaire (CEA), l'antigène tumoral 125 (CA125), l'AFP, SCC, CA19-9, CA242, NSE, Cyfa21-1, CA15-3, et PSA total T-PSA.

14. Procédé selon l'une quelconque des revendications 10 à 13, dans lequel le film comprend en outre un réseau d'éléments de liaison sur le film, les éléments de liaison se liant à l'analyte.

15. Procédé selon la revendication 14, dans lequel les éléments de liaison sont une pluralité d'antigènes pour la détection d'anticorps dans un échantillon, les antigènes se trouvant sur une surface du film ; et
le procédé comprenant :
la mise en contact de la pluralité d'antigènes avec un échantillon et un ou plusieurs marqueurs pour la détection desdits un ou plusieurs anticorps liés à un antigène de la pluralité d'antigènes, chaque marqueur comprenant un fluorophore ; et
la détection desdits un ou plusieurs anticorps liés par détection d'un signal fluorescent du fluorophore, l'intensité du signal fluorescent étant augmentée par rapport au signal fluorescent du fluorophore en l'absence de film.

16. Procédé selon la revendication 15, dans lequel lesdits un ou plusieurs anticorps sont des anticorps IgG, et lesdits un ou plusieurs marqueurs sont des anticorps anti-IgG ;
ou dans lequel (i) lesdits un ou plusieurs anticorps comprennent un ou plusieurs sous-types d'anticorps sélectionnés parmi IgG, IgM, IgA, et IgE ; (ii) lesdits un ou plusieurs marqueurs comprennent une pluralité d'anticorps marqueurs correspondants sélectionnés parmi des anticorps anti-IgG, anti-IgM, anti-IgA et anti-IgE ; (iii) le fluorophore d'un anticorps marqueur correspondant à un sous-type est différent du fluorophore correspondant à tout autre sous-type, les fluorophores étant sélectionnés par exemple parmi Cy3, Cy5, IR680 et IR800 ; et (iv) le procédé comprenant en outre une identification ou une quantification des différents sous-types en fonction du signal fluorescent.
